# EUROPÄISCHE PATENTSCHRIFT

(11) **EP 2 944 380 B1**
(45) Veröffentlichungstag und Bekanntmachung des Hinweises auf die Patenterteilung: **19.07.2017**
(21) Anmeldenummer: 15160736.3
(22) Anmeldetag: 25.03.2015
(51) Int. Cl.: B05C 17/005, B01F 15/00, B05C 17/01, B01F 3/10, B01F 7/00, B01F 13/00, B01F 15/02, A61B 17/88

(54) **VORRICHTUNG UND VERFAHREN ZUM MISCHEN EINES MEHRKOMPONENTENZEMENTS**
DEVICE AND METHOD FOR MIXING A MULTIPLE COMPONENT CEMENT
DISPOSITIF ET PROCÉDÉ DESTINÉS AU MÉLANGE DE CIMENT À PLUSIEURS COMPOSANTS

(30) Priorität: 15.05.2014 DE 102014106873
(43) Veröffentlichungstag der Anmeldung: 18.11.2015
(73) Patentinhaber: Heraeus Medical GmbH, 61273 Wehrheim (DE)
(72) Erfinder: Vogt, Sebastian, 99092 Erfurt (DE)
(74) Vertreter: Heraeus IP

(56) Entgegenhaltungen:
- EP-A1- 2 324 792
- DE-A1- 3 307 558
- DE-A1- 3 738 960
- DE-U1- 8 907 735
- DE-U1-202009 017 678
- US-A- 3 117 696
- US-A- 3 774 816

## Beschreibung

Die Erfindung betrifft eine manuell antreibbare Vorrichtung zum Mischen eines pastösen Mischguts aus wenigstens zwei fließfähigen Ausgangskomponenten, insbesondere zum Mischen eines medizinischen Zements, und gegebenenfalls zur Lagerung der Ausgangskomponenten.

Die Erfindung betrifft ferner ein Verfahren zum Mischen von fluiden Ausgangskomponenten und gegebenenfalls auch zum Austragen eines Mischguts, insbesondere zum Mischen eines medizinischen Zements.

Gegenstand der Erfindung ist insbesondere auch eine Vorrichtung zur Lagerung und Vermischung von Polymethylmethacrylat-Knochenzement (PMMA-Knochenzement), der vor der Vermischung, während der Lagerung, aus einer flüssigen oder pastenförmigen ersten Komponente und dazu separaten pulverförmigen oder pastenförmigen zweiten Komponente besteht, sowie ein Verfahren zum Vermischen der beiden Komponenten und gegebenenfalls zum Applizieren des gemischten PMMA-Knochenzements sowie, wenn gewünscht auch einem zusätzlichen pharmazeutischen Wirkstoff, der einer der Ausgangskomponenten oder beiden Ausgangskomponenten beigefügt werden kann oder zugesetzt ist.

In der Medizin werden seit Jahrzehnten Polymethylmethacrylat-Knochenzemente (PMMA-Knochenzemente) zur dauerhaften mechanischen Fixierung von Totalgelenkendoprothesen eingesetzt. Diese basieren auf Pulver-Flüssigkeits-Systemen, wobei Methylmethacrylat üblicherweise als Monomer verwendet wird. Es wurden in jüngster Zeit auch Polymethylmethacrylat-Knochenzemente vorgeschlagen, die auf der Verwendung von Zementpasten beruhen (DE 10 2007 050 762 B3, DE 10 2008 030 312 A1, DE 10 2007 052 116 A1). Bei diesen Knochenzementen werden zwei Zementpasten separat in geeigneten Kartuschen gelagert. Solche Kartuschen werden häufig auch als 2-KomponentenKartuschen (auch 2K-Kartuschen) bezeichnet. Diese enthalten jeweils neben mindestens einem Monomer und geeigneten Polymeren Bestandteile eines Redoxinitiatorsystems. Solche Ausgangskomponenten sind auch in erfindungsgemäßen Vorrichtungen und bei erfindungsgemäßen Verfahren bevorzugt einsetzbar und anwendbar.

Das am häufigsten in Polymethylmethacrylat-Knochenzementen verwendete Monomer ist Methylmethacrylat. Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Bestandteile der Redoxinitiatorsysteme zusammenwirken. Deshalb werden die Bestandteile des Redoxinitiatorsystems in den separaten Ausgangskomponenten so angeordnet, dass diese keine radikalische Polymerisation auslösen können. Die Ausgangskomponenten sind bei geeigneter Zusammensetzung lagerstabil. Erst bei Vermischung der beiden Ausgangskomponenten zu einem Zementteig reagieren die zuvor getrennt in beiden Pasten gelagerten Bestandteile des Redoxinitiatorsystems, wobei Radikale gebildet werden, welche die radikalische Polymerisation des wenigstens einen Monomers auslösen. Die radikalische Polymerisation führt dann unter Verbrauch des Monomers zu Bildung von Polymeren, wobei der Zementteig aushärtet. Zur Vermischung der Ausgangskomponenten werden üblicherweise statische Mischer eingesetzt, die dazu an die mit den Ausgangskomponenten gefüllten Zwei-Komponentenkartuschen angebracht werden.

Beim Auspressen der beiden pastenförmigen Ausgangskomponenten aus den Kartuschen werden diese durch einen statischen Mischer gedrückt. Der Auspress- und der Vermischungsvorgang erfolgen dadurch gleichzeitig. Zur Vermischung der Ausgangskomponenten im statischen Mischer ist eine hohe Auspresskraft notwendig, weil der Druckabfall innerhalb des statischen Mischers an den Mischelementen sehr hoch ist. Dadurch ist es notwendig, leistungsfähige pneumatische oder mechanische Auspressvorrichtungen einzusetzen, um einen Austrag und eine optimale Vermischung der pastenförmigen Ausgangskomponenten zu erreichen. Diese pneumatischen oder auch mechanischen Auspressvorrichtungen sind technisch aufwendig und teuer. Kostengünstig sind dagegen die, bisher bei den auf Pulver-Flüssigkeits-Systemen beruhenden Polymethylmethacrylat-Knochenzementen üblichen, manuell betriebenen Auspresspistolen, welche sich für diese Zemente eignen, jedoch für die Auspressung und Vermischung von Knochenzementpasten unter Verwendung von statischen Mischern nicht ausreichend leistungsfähig sind.

Bei ungleichen Volumina beziehungsweise stark unterschiedlichen Mischungsverhältnissen (2:1 und mehr) der zu mischenden Ausgangskomponenten sind sehr viele Mischwendel durch die größeren Volumenverhältnissen notwendig. Je größer die Anzahl der benötigten Mischwendel ist, umso größer ist auch der Druckabfall im statischen Mischer beim Mischprozess. Es muss eine pastenförmige Komponente vorhanden sein, die zweite Komponente kann flüssig oder pulverförmig oder ebenfalls pastenförmig sein. Die Komponenten beziehungsweise Pasten müssen mit sehr großer Kraft durch den statischen Mischer gepresst werden. Bei manuell betriebenen Auspressvorrichtungen ist naturgemäß die maximal mögliche Auspresskraft beschränkt.

In der Klebstoff- und Dichtungsmittelindustrie hat sich seit Jahren das System Semkit^{®} bewährt. Dabei wird in einem Lagerbehälter eine Paste gelagert. In einem Rührstab ist eine zweite flüssige Komponente enthalten, die durch ein im Rührstab integriertes Ventil von der Paste getrennt ist. Bei Betätigen des Ventils läuft die Flüssigkeit in die Paste, die dann manuell gemischt werden kann.

Nachteilig ist an einem solchen System, dass mehrere Schritte notwendig sind, um das Mischgut, insbesondere den medizinischen Zement, einsatzfähig zu machen. Nachteilig ist an den bekannten Systemen ferner, dass keine einfache manuelle Bedienung der Vorrichtungen möglich ist, durch die die Mischung und das Austragen der pastösen Massen möglich wären. Dies ist im oft hektischen und stressigen Betrieb während einer Operation am Patienten, also im medizinischen Einsatz, besonders nachteilig. Eine einfacher und manuell zu bedienende Vorrichtung zum Mischen der Ausgangskomponenten wäre dementsprechend wünschenswert. Bei Systemen, die mit Druckluft und/oder einem elektrischen Motor betrieben werden, sind kostenintensive Bauteile notwendig, das System kann dann nicht mehr als hygienescher Einmalartikel verwendet werden.

Aus der DE 89 07 735 U1 ist eine Vorrichtung zum Auspressen von Kartuschen mit einem hydraulischen Antrieb bekannt, der über einen Hebel bedient wird. Durch das wiederholte Bedienen des Hebels werden zudem Mischflügel in einem Mischraum hin- und hergedreht. DE 89 07 735 U1 offenbart eine Vorrichtung entsprechend dem Oberbegriff des Anspruchs 1 und ein Verfahren entsprechend dem Oberbegriff des Anspruchs 18. Für pastenförmige Polymethylmethacrylat-Knochenzemente sind formstabile, feste Kartuschen notwendig, weil der relativ zähe pastenförmige Polymethylmethacrylat-Knochenzementteig und/oder die pastenförmigen Ausgangskomponenten nur mit großen Auspresskräften aus Lagerbehältern beziehungsweise Kartuschen ausgepresst werden können. Weiterhin ist es für Knochenzemente nicht möglich, Mischsysteme zu verwenden, bei dem ein unerwünschter Austritt geringer Pastenmengen auftritt und bei dem die Möglichkeit besteht, dass Luft in die Paste gezogen wird. Dadurch würde einerseits die Sauberkeit im Operationssaal beeinträchtigt und andererseits wird durch Eintrag von Luft der Zementteig mechanisch geschwächt, weil Luftblasen im ausgehärteten Zement als Rissansatzstellen wirken und damit die Festigkeit des ausgehärteten Knochenzements herabsetzen. Dadurch kann das Semkit^{®}-System nicht für pastenförmige Polymethylmethacrylat-Knochenzemente eingesetzt werden.

Die Aufgabe der Erfindung besteht also darin, die Nachteile des Stands der Technik zu überwinden. Insbesondere soll eine kostengünstig zu fertigende und zuverlässig funktionierende Vorrichtung zum Mischen eines pastösen Mischguts, insbesondere eines medizinischen Zements, aus wenigstens zwei fluiden Ausgangskomponenten und gegebenenfalls zur Lagerung der Ausgangskomponenten, und ein Verfahren zum Mischen des pastösen Mischguts gefunden werden, bei dem eine möglichst einfache manuelle Bedienung zum Mischen von separat in Kartuschen aufbewahrten fluiden Ausgangskomponenten, insbesondere aus hochviskosen beziehungsweise zähen Pasten, angewendet werden kann, ohne dass eine externe oder zusätzliche Energiequelle verwendet werden muss und ohne dass Lufteinschlüsse in dem Mischgut entstehen.

Insbesondere soll eine einfache und kostengünstige Vorrichtung zur Lagerung und Vermischung von Polymethylmethacrylat-Knochenzement entwickelt werden, mit der zumindest eine Ausgangskomponente unter Luftausschluss gelagert werden kann, wobei mit der Vorrichtung eine Vermischung der Ausgangskomponenten und bevorzugt auch ein Austragen des Zementteigs erfolgen können soll.

Die Hauptkomponente des Polymethylmethacrylat-Knochenzements als Mischgut soll eine Zementpaste sein und die zweite Komponente kann ebenfalls pastenförmig sein oder auch in Form eines Pulvers vorliegen. Die beiden Ausgangskomponenten des Knochenzements sollen getrennt gelagert werden können und durch Anwendung der Vorrichtung sicher zusammengeführt werden können.

Die Aufgaben der Erfindung werden gelöst durch eine manuell antreibbare Vorrichtung zum Mischen eines pastösen Mischguts aus wenigstens zwei fluiden Ausgangskomponenten, insbesondere zum Mischen eines medizinischen Zements, aufweisend:
A) zumindest zwei Kartuschen enthaltend die fluiden Ausgangskomponenten oder zumindest zwei Anschlüsse für zumindest zwei Kartuschen enthaltend die fluiden Ausgangskomponenten, wobei in den Kartuschen Förderkolben zum Austreiben der Ausgangskomponenten aus den Kartuschen vorgesehen sind;
B) einen Mischraum zum Mischen der Ausgangskomponenten, der mit den Kartuschen oder den Anschlüssen für die Kartuschen verbunden ist, so dass beim Austreiben der Ausgangskomponenten aus den Kartuschen mit den Förderkolben die Ausgangskomponenten in den Mischraum geleitet werden, wobei in dem Mischraum Mischflügel vorgesehen sind, die in dem Mischraum drehbar gelagert sind;
C) einen um einen Drehpunkt drehbaren und manuell bedienbaren Hebel zum Bedienen der Vorrichtung oder einen in Längsrichtung verschiebbaren und manuell bedienbaren Stift zum Bedienen der Vorrichtung;
D) ein Getriebe, das mit dem Hebel oder dem Stift verbunden ist, wobei das Getriebe mit den drehbaren Mischflügeln in dem Mischraum verbunden ist, so dass durch eine Bewegung des Hebels oder des Stifts die Mischflügel in dem Mischraum vermittelt über das Getriebe drehbar sind, wobei in der Verbindung von dem Getriebe zu den Mischflügeln oder im Getriebe eine Kupplung vorgesehen ist, die eine Drehung der Mischflügel in einer Drehrichtung ermöglicht und in der entgegengesetzten Drehrichtung verhindert oder reduziert; und
E) eine Einrichtung zum Vortreiben der Förderkolben in den Kartuschen, die mit dem Hebel oder dem Stift verbunden ist, so dass bei einer Bewegung des Hebels oder des Stifts die Förderkolben in den Kartuschen vorzutreiben sind, so dass die Bewegung des Hebels oder des Stifts die Drehung der Mischflügel in dem Mischraum und die Bewegung der Förderkolben in die Kartuschen hinein antreibt.

Bei anschließbaren Kartuschen ist die Einrichtung selbstverständlich zum Vortreiben der Förderkolben in den angeschlossenen Kartuschen geeignet, wobei die Einrichtung mit dem Hebel oder dem Stift verbunden ist, so dass bei einer Bewegung des Hebels oder des Stifts die Förderkolben in den angeschlossenen Kartuschen vorzutreiben sind.

Die Vorrichtung ist insbesondere und erfindungsgemäß besonders bevorzugt eine Zementierpistole.

Die Ausgangskomponenten für das Mischgut, insbesondere für den Knochenzement, sind erfindungsgemäß bereits in den Kartuschen enthalten.

Die Vorrichtung ist erfindungsgemäß bevorzugt auch zum Lagern der Ausgangskomponenten geeignet, insbesondere dann, wenn die Kartuschen in die Vorrichtung eingesetzt sind oder die Kartuschen ein fester Teil der Vorrichtung sind.

Als fluide Ausgangskomponenten kommen flüssige, pastöse also pastenförmige und fließfähige pulverförmige Ausgangskomponenten in Frage. Wenigstens eine der Ausgangskomponenten ist pastös oder flüssig, um ein pastöses Mischgut zu erhalten. Bevorzugt sind beide Ausgangskomponenten pastös.

Besonders bevorzugt ist das Mischgut ein Knochenzement, insbesondere ein PMMA-Knochenzement.

Bevorzugt ist in den Kartuschen gegenüberliegend zu den Förderkolben zum Austreiben der Ausgangskomponenten aus den Kartuschen jeweils eine gegenüberliegende Öffnung in jeder Kartusche vorgesehen, durch die die Ausgangskomponenten aus den Kartuschen in den Mischraum zu leiten sind.

Ebenfalls bevorzugt kann vorgesehen sein, dass die Bewegung des Hebels oder des Stifts, durch die über das Getriebe die Mischflügel in dem Mischraum drehbar sind und durch die die Förderkolben in den Kartuschen vorgetrieben werden, eine manuell angetriebene Bewegung ist.

Auch kann bevorzugt an dem Hebel oder dem Stift ein Rückstellelement, wie eine Druckfeder angeordnet sein, mit dem der Hebel oder der Stift nach der manuellen Bewegung des Hebels oder des Stifts wieder in seine Ausgangssituation überführt wird. Besonders bevorzugt wird bei der manuellen Bewegung des Hebels beziehungsweise des Stifts das Rückstellelement gespannt oder komprimiert.

Bei erfindungsgemäßen Vorrichtungen kann vorgesehen sein, dass die Mischflügel gegen die Achse, um die sich die Mischflügel im Mischraum drehen, im gleichen Sinn geneigt sind, so dass durch die Drehung der Mischflügel im Mischraum ein Vortrieb des Mischguts im Mischraum erfolgt, wobei bevorzugt die Mischflügel um den gleichen Winkel gegen die Achse geneigt sind.

Die Mischflügel können bevorzugt alle gegen die Achse geneigt sein, müssen aber nicht alle gegen die Achse geneigt sein. Im gleichen Sinn geneigt bedeutet dabei, dass die Mischflügel bezogen auf die Achse, um die sich die Mischflügel drehen, im gleichen Winkel geneigt sind. Gegenüberliegende Mischflügel sind dadurch nicht spiegelsymmetrisch angeordnet sondern rotationssymmetrisch bezogen auf diese Achse. Die Mischflügel bilden dadurch einen Propeller oder eine Turbinenschaufelkonfiguration.

Hierdurch sind die Mischflügel gut sowohl zum Mischen des Mischguts beziehungsweise zum Mischen der beiden Ausgangskomponenten als auch zum Vortreiben des Mischguts in dem Mischraum einsetzbar. Dies ermöglicht auch die Verwendung von zähen beziehungsweise hochviskosen Pasten als Ausgangskomponenten in der durch Handkraft angetriebenen Vorrichtung.

Bevorzugte Ausgestaltungen der erfindungsgemäßen Vorrichtung können sich dadurch auszeichnen, dass das Getriebe kraftschlüssig und/oder formschlüssig ist.

Solche Getriebe sind besonders gut zur Übertragung der manuell ausgeübten Kräfte geeignet. Das Getriebe kann bevorzugt ein geringes oder gar kein Spiel haben.

Mit einer Weiterbildung der vorliegenden Erfindung wird auch vorgeschlagen, dass das Getriebe wenigstens eine Zahnscheibe, wenigstens eine Reibscheibe, wenigstens ein Zahnrad, wenigstens ein Schneckenrad und/oder wenigstens ein Reibrad aufweist, über das oder die die Kraft beziehungsweise das Drehmoment übertragen wird. Bevorzugt weist das Getriebe wenigstens eine Kegelzahnscheibe, wenigstens eine Kegelreibscheibe, wenigstens ein Kegelzahnrad und/oder wenigstens ein Kegelreibrad zur Veränderung der Drehrichtung auf.

Diese Bauteile sind einfach und kostengünstig auch aus Kunststoff mit der notwendigen Stabilität zu fertigen. Die Verwendung von kegelförmig ausgestalteten Kraftübertragungselementen hat den Vorteil, dass die Richtung des übertragenen Drehmoments in dem Getriebe geändert werden kann.

Bevorzugte Ausgestaltungen der vorliegenden Erfindung können vorsehen, dass das Getriebe ein Zahnradgetriebe, ein Reibradgetriebe, ein Kegelradgetriebe, ein Kronenradgetriebe, ein Schneckengetriebe oder eine Kombination solcher Getriebe ist, bevorzugt zusätzlich aufweisend ein Stirnradgetriebe.

Reibradgetriebe werden häufig auch als Wälzkörpergetriebe bezeichnet. Der Antrieb kann sowohl über die Stirnseite des Zahnrads oder Reibrads (normaler Aufbau) oder über eine randseitige Seitenfläche des Zahnrads oder des Reibrads (Seitenflächenzahnung oder Seitenflächenreibung) erfolgen. Die genannten Getriebe sind einfach und kostengünstig im Aufbau, für die notwendige Kraftübertragung und Drehmomentübertragung geeignet und unanfällig im Betrieb.

Gemäß einer besonders bevorzugten Ausgestaltung der vorliegenden Erfindung kann vorgesehen sein, dass die Mischflügel auf einer drehbar gelagerten Drehachse (im Mischraum) angeordnet sind, wobei die Drehachse sich aus dem Mischraum heraus erstreckt, bevorzugt über eine Dichtung aus dem Mischraum heraus erstreckt, und die Drehachse mit dem Getriebe verbunden ist, so dass durch eine Bewegung des Hebels oder des Stifts die Drehachse mit den Mischflügeln in dem Mischraum drehbar ist.

Dies hat den Vorteil, dass einerseits der Aufbau besonders einfach ist und zusätzlich ist die Mittelachse des Mischraums durch die Drehachse ausgefüllt, so dass dort keine schlecht gemischten Teile des Mischguts durchgedrückt werden können.

Ferner ist vorgesehen, dass in der Verbindung von dem Getriebe zu den Mischflügeln, insbesondere in der drehbar gelagerten Drehachse, oder im Getriebe eine Kupplung vorgesehen ist, die eine Drehung der Mischflügel in einer Drehrichtung ermöglicht und in der entgegengesetzten Drehrichtung verhindert oder reduziert.

Hierdurch wird erreicht, dass sich die Mischflügel nur in einer Richtung drehen oder hauptsächlich in einer Richtung drehen. Wenn die Mischflügel auch zum Vortrieb des Mischguts genutzt werden, wird dadurch verhindert, dass sich die Mischflügel bei der Rückwärtsbewegung des Hebels oder des Stifts in umgekehrter Richtung drehen und dadurch das Mischgut ungewollt zurück in Richtung der Kartuschen gefördert wird.

Dabei kann vorgesehen sein, dass an dem Getriebe oder in dem Getriebe eine Freilaufkupplung vorgesehen ist oder in der Verbindung von dem Getriebe zu den Mischflügeln, insbesondere in der drehbar gelagerten Drehachse, eine gefederte Drehkupplung vorgesehen ist.

Solche gefederten Drehkupplungen, die vorliegend ebenfalls als Freilaufkupplungen bezeichnet werden, sind beispielsweise aus Kugelschreibern bekannt und sehr kostengünstig in der Herstellung, gleichzeitig in Ihrer Funktion aber für die erfindungsgemäße Anwendung ausreichend. Bei solchen gefederten Drehkupplungen greift eine gezahnte Kreisfläche in ein passendes Gegenstück, wobei die beiden Kreisflächen mit einer elastischen Feder aufeinander gedrückt werden. Die Zahnung hat in einer Drehrichtung eine steile Flanke, bevorzugt eine senkrechte Flanke, und in der anderen Drehrichtung eine flache Flanke. Bei einer Drehung der angetriebenen Kreisfläche in Richtung der steilen Flanke dreht sich die andere Kreisfläche mit, da die steilen Flanken ineinander greifen. Bei einer gegenläufigen Drehung rutschen die flachen Flanken übereinander weg und die andere Kreisfläche dreht sich aufgrund des Schlupfs nicht mit. So wird erreicht, dass die Drehung der Achse nur in einer Richtung bis zu den Mischflügeln weitergeleitet wird.

Hierdurch wird auf einfache und kostengünstige Weise erreicht, dass sich die Mischflügel nur in einer Richtung drehen und nicht bei einer Rückstellung oder Rückbewegung des Hebels oder des Stifts in die entgegengesetzte Richtung drehen können und dabei das Mischgut im Mischraum ungewollt in Richtung der Kartuschen drücken.

Es kann erfindungsgemäß vorgesehen sein, dass die Einrichtung zum Vortreiben der Förderkolben in den Kartuschen eine mechanische Vortriebseinrichtung ist. Hierdurch wird ein einfacher Aufbau der Vorrichtung erreicht.

Mit einer Weiterbildung der vorliegenden Erfindung wird vorgeschlagen, dass die Einrichtung zum Vortreiben der Förderkolben in den Kartuschen eine Klemmstange mit einer kippbaren Verkantungsöffnung oder eine Zahnstange mit einer unidirektional wirkenden Rastung ist, wobei die Verkantungsöffnung oder die Rastung mit dem Hebel verbunden ist oder die Verkantungsöffnung oder die Rastung mit dem Stift verbunden ist, insbesondere die Verkantungsöffnung oder die Rastung mit dem Stift über einen Hebel verbunden ist.

Die Einrichtung zum Vortreiben der Förderkolben in den Kartuschen kann auch mit einer Spindel aufgebaut werden. Durch die genannten Maßnahmen wird erreicht, dass ein schrittweiser Vortrieb der Förderkolben durch mehrmaliges Bedienen beziehungsweise mehrmaligen Hub des Hebels oder des Stifts ermöglicht wird. Hierdurch kann erreicht werden, dass auch größere Mengen des Mischguts, wie es beispielsweise bei Knochenzementen notwendig ist, aus zähen oder hochviskosen Pasten manuell gemischt werden kann. Solche Verkantungsöffnungen sind beispielsweise bei Kartuschenpistolen für Silikon bekannt.

Um auch eine längere Lagerung der Ausgangskomponenten in der Vorrichtung zu ermöglichen, kann vorgesehen sein, dass in der Verbindung zwischen dem Mischraum und den Kartuschen oder den Anschlüssen für die Kartuschen zumindest ein manuell zu öffnendes Ventilelement angeordnet ist oder manuell zu öffnende Verschlüsse angeordnet sind.

Dadurch kann die Einrichtung auch gut zum Lagern der Ausgangskomponenten eingesetzt werden, da die Ausgangskomponenten in den Kartuschen nach außen durch die Kartuschenwände, auf einer Seite durch die Förderkolben und auf der gegenüberliegenden Seite durch die Verschlüsse oder das Ventilelement geschlossen sind. Dadurch bleibt der Inhalt auch bei Lagerung steril und trocknet nicht so schnell aus.

Bevorzugt wird als Ventilelement ein axial drehbares an den zylindrischen Innenwänden des Mischraums anliegendes zylindrisches Rohrstück verwendet, wobei in der Mantelfläche des Rohrstücks zwei Durchführungen angeordnet sind, wobei sich die Durchführungen durch Drehung des Rohrstücks in dem Mischraum mit den Verbindungen zu den Kartuschen in Überdeckung bringen lassen. Durch die Überdeckung der Durchführungen mit den Verbindungen zu den Kartuschen ist das Ventilelement geöffnet. Wenn die Durchführungen mit Verbindungsöffnungen zu den Kartuschen in Überdeckung gebracht sind, ist das Ventilelement in einer geöffneten Stellung. Dieser Aufbau ist einfach und kostengünstig aus Kunststoff herstellbar und unanfällig in der Bedienung. Besonders bevorzugt ist an dem Rohrstück ein Bedienungshebel befestigt, der sich durch eine Öffnung in der Wandung des Mischraums erstreckt, insbesondere sich durch einen radialen Schlitz in der Wandung des Mischraums erstreckt. Dadurch kann das Rohrstück manuell in dem Mischraum gedreht und damit geöffnet und, wenn gewollt, auch wieder geschlossen werden.

Eine besonders leicht zu bedienende erfindungsgemäße Vorrichtung kann dadurch erreicht werden, dass die Vorrichtung einen Griff zum Halten der Vorrichtung mit einer Hand aufweist, wobei der Hebel oder der Stift oder ein Bedienungselement zur manuellen Bedienung des Hebels oder des Stifts derart im Bereich des Griffs angeordnet ist, dass der Hebel oder der Stift oder das Bedienungselement mit der gleichen Hand bedienbar ist, mit der die Vorrichtung am Griff gehalten wird.

Hierdurch kann die Vorrichtung mit einer Hand bedient werden. Dies vereinfacht die manuelle Bedienung.

Bevorzugte erfindungsgemäße Vorrichtungen können sich dadurch auszeichnen, dass der Mischraum auf der der Verbindung zu den Kartuschen gegenüberliegenden Seite eine Austragsöffnung zum Applizieren des Mischguts aufweist, wobei bevorzugt an der Austragsöffnung ein Applikationsrohr angeschlossen oder anschließbar ist.

Hierdurch kann das Mischgut direkt mit der Vorrichtung appliziert beziehungsweise ausgetragen und angewendet werden.

Mit einer Weiterbildung der vorliegenden Erfindung wird auch vorgeschlagen, dass in dem Mischraum statische Scherelemente zum Durchmischen des Mischguts angeordnet sind, wobei bevorzugt die Scherelemente den Fluss des Mischguts durch den Mischraum zerschneiden und/oder die Scherelemente in axialer Richtung, bezogen auf die Achse, um die sich die Mischflügel drehen, zwischen den Mischflügeln angeordnet sind.

Durch die Verwendung der Scherelemente, die das Mischgut in Förderrichtung durchschneiden, wird eine stärkere Durchmischung des Mischguts erzielt.

Mit einer Weiterentwicklung der Erfindung wird auch vorgeschlagen, dass an dem Hebel ein Kreisbogen oder ein Kreisbogenabschnitt angeordnet ist, auf dessen Stirnseite oder auf dessen Seitenfläche eine Antriebszahnreihe oder eine Reibfläche angeordnet ist, mit der ein Zahnrad oder Reibrad des Getriebes antreibbar ist oder an dem Stift eine Zahnung oder eine Reibfläche angeordnet ist, mit der ein Zahnrad oder Reibrad des Getriebes antreibbar ist.

Die Antriebszahnreihe oder Reibfläche kann auch gegenüber der Stirnseite oder der Seitenfläche des Kreisbogens oder des Kreisbogenabschnitt geneigt sein. Zudem oder alternativ kann dabei das Zahnrad oder Reibrad des Getriebes, das von der Antriebszahnreihe oder Reibfläche angetrieben wird, ein Kegelrad sein.

Durch die Verwendung eines Kreisbogens mit dem Hebel, kann die Kippbewegung des Hebels unmittelbar in eine Drehbewegung des Kreisbogens umgesetzt werden, mit der das Getriebe angetrieben wird. Dieser Aufbau ist sowohl kostengünstig zu realisieren, als auch einfach und unanfällig für Fehlfunktionen.

Besonders vorteilhafte Ausgestaltungen der Erfindung können sich dadurch auszeichnen, dass das Getriebe eine derartige Übersetzung aufweist, dass bei einer vollständigen Bewegung des Hebels oder des Stifts die Mischflügel wenigstens 2 Umdrehungen im Mischraum vollführen, bevorzugt zwischen 2 und 10 Umdrehungen im Mischraum vollführen, besonders bevorzugt zwischen 2,5 und 5 Umdrehungen im Mischraum vollführen.

Wenn diese Übersetzungen gewählt werden, kann mit einem Hub eine ausreichende Durchmischung und ein ausreichender Vortrieb auf das Mischgut in dem Mischraum auch bei hochviskosen und sehr zähen Pasten als Ausgangskomponenten ausgeübt werden, ohne dass die Kraft der Hand eines normalen Benutzers nicht mehr ausreichen würde, um die Vorrichtung manuell, insbesondere mit einer Hand zu bedienen.

Des Weiteren kann vorgesehen sein, dass die Einrichtung zum Vortreiben der Förderkolben in den Kartuschen derart mit dem Hebel oder dem Stift verbunden ist, dass bei einer vollständigen Bewegung des Hebels oder des Stifts die Förderkolben um wenigstens 1 mm in den Kartuschen vorgetrieben werden, bevorzugt zwischen 2 mm und 12 mm in den Kartuschen vorgetrieben werden, besonders bevorzugt zwischen 5 mm und 7 mm in den Kartuschen vorgetrieben werden.

Bei diesem Vortrieb, der bevorzugt passend zu der Drehgeschwindigkeit der Mischflügel in dem Mischraum gewählt wird, wird erreicht, dass ein Austragen der Ausgangskomponenten aus den Kartuschen auch bei zähen beziehungsweise hochviskosen Ausgangskomponenten möglich ist.

Bei besonders bevorzugten Ausführungen der Erfindung kann auch vorgesehen sein, dass die Einrichtung zum Vortreiben der Förderkolben in den Kartuschen derart mit dem Hebel oder dem Stift verbunden ist, dass bei 5 bis 25 vollständigen Bewegungen des Hebels oder des Stifts die Förderkolben die Ausgangskomponenten zu wenigstens 90% aus den Kartuschen austreiben, bevorzugt dass bei 10 bis 20 vollständigen Bewegungen des Hebels oder des Stifts die Förderkolben die Ausgangskomponenten zu wenigstens 90% aus den Kartuschen austreiben.

Hierdurch wird, insbesondere für die Anwendung der Vorrichtung zum Mischen und Applizieren von Knochenzement, eine gute Brauchbarkeit der Vorrichtung erreicht. Die angegebenen Bewegungszyklen können ohne zu große Mühe durchgeführt werden, ohne dass der Arm beziehungsweise die Hand des Benutzers ermüdet aber gleichzeitig so, dass jede einzelne Bewegung, das heißt jeder einzelne Hub des Hebels oder des Stifts, nicht zu viel Kraft erfordert.

Die Vorrichtung besteht bevorzugt bis auf Rückstellelemente und/oder Federelemente aus Kunststoff, besonders bevorzugt aus Thermoplast. Ganz besonders bevorzugt bestehen auch die Rückstellelemente und/oder Federelemente aus Kunststoff.

Erfindungsgemäß ist die Vorrichtung von einem Gehäuse umschlossen, so dass ein Eingriff in sich während der Anwendung bewegende Teile der Vorrichtung außer dem Hebel oder dem Stift verhindert ist. Der Hebel oder der Stift werden zur Anwendung der Vorrichtung bedient und müssen daher für den Anwender zugänglich sein.

Die der Erfindung zugrundeliegenden Aufgaben werden auch gelöst durch ein Verfahren zum Mischen von fluiden Ausgangskomponenten und gegebenenfalls auch zum Austragen eines pastösen Mischguts, insbesondere zum Mischen eines medizinischen Zements, vorzugsweise mit einer Vorrichtung nach einem der vorangehenden Ansprüche, dadurch gekennzeichnet, dass
A) ein Hebel gekippt wird oder ein Stift in Längsrichtung bewegt wird,
B) durch die Kraft der Bewegung des Hebels oder des Stifts Förderkolben in wenigstens zwei Kartuschen vorangetrieben werden und die in den Kartuschen enthaltenen Ausgangskomponenten mit Hilfe der Förderkolben in einen Mischraum gepresst werden, und
C) durch die Kraft der Bewegung des Hebels oder des Stifts Mischflügel in dem Mischraum gedreht werden, wobei die Übertragung der Kraft des Hebels oder des Stifts zur Drehung der Mischflügel über ein Getriebe erfolgt, wobei nach der Bewegung des Hebels oder des Stifts dieser durch ein Rückstellelement wieder in die Ausgangsposition überführt wird, wobei bei dieser Überführung in die Ausgangsposition die Förderkolben nicht bewegt werden und die Mischflügel nicht gedreht werden.

Dabei kann vorgesehen sein, dass durch die Drehung der Mischflügel in dem Mischraum die Ausgangskomponenten in dem Mischraum durchmischt werden und in Richtung einer Austragsöffnung gefördert werden.

Durch diese gleichzeitige Mischung und Förderung können auch hochviskose und zähe Pasten als Ausgangskomponenten für das Mischgut verwendet werden. Gleichzeitig ist noch eine manuelle Bedienung der Vorrichtung beziehungsweise ein manueller Antrieb des Hebels oder des Stifts ausreichend, um die Mischflügel und die Förderkolben in der Vorrichtung anzutreiben.

Es ist vorgesehen, dass nach der Bewegung des Hebels oder des Stifts dieser durch ein Rückstellelement wieder in die Ausgangsposition überführt wird, wobei bei dieser Überführung in die Ausgangsposition die Förderkolben nicht bewegt werden und die Mischflügel nicht gedreht werden.

Hierdurch kann die Vorrichtung durch mehrfachen Hub des Hebels oder des Stifts angewendet werden, wodurch sich die aufzuwendende Kraft und die erzeugte Menge Mischgut pro Hub einstellen lässt beziehungsweise in kleineren Portionen erfolgen kann.

Es kann auch vorgesehen sein, dass das Getriebe über einen Zahnkranz oder Reibkranz am Hebel oder über eine Zahnreihe oder eine Reibfläche am Stift angetrieben wird.

Mit der Erfindung wird auch vorgeschlagen, dass die Bewegung des Stifts oder des Hebels mehrmals wiederholt wird, bevorzugt zwischen 5 und 25 mal wiederholt wird, um die Kartuschen zu wenigstens 90% zu entleeren. Besonders bevorzugt wird die Bewegung des Stifts oder des Hebels 10 bis 15 mal wiederholt, um die Kartuschen zu wenigstens 90% zu entleeren

Auch kann vorgesehen sein, dass das Getriebe einen vollständigen Hub des Hebels oder des Stifts in wenigstens 2 Umdrehungen der Mischflügel in dem Mischraum übersetzt, bevorzugt in 2,5 bis 5 Umdrehungen der Mischflügel in dem Mischraum übersetzt.

Vor dem erstmaligen Bedienen des Hebels oder des Stifts kann vorgesehen sein, dass die Kartuschen zum Mischraum geöffnet werden, bevorzugt durch manuelles Bedienen eines Ventilelements. Die Ausgangskomponenten können dann zuvor über längere Zeiträume in den Kartuschen gelagert werden.

Der Erfindung liegt die überraschende Erkenntnis zugrunde, dass es durch die Verbindung des manuell zu bedienenden Hebels oder Stifts sowohl mit den drehbaren Mischflügeln als auch mit der Einrichtung zum Vortreiben der Förderkolben in den Kartuschen gelingt, mit einem Hub des Hebels oder des Stifts oder bevorzugt mit mehreren Hüben des Hebels oder des Stifts die Ausgangskomponenten aus den Kartuschen auszupressen und in dem Mischraum gleichzeitig eine aktive Vermischung der Ausgangskomponenten zu erreichen. Bei geeigneter Stellung der Mischflügel, indem die Mischflügel gegen die Achse geneigt sind, um die sich die Mischflügel drehen, und bei geeigneter Drehrichtung der Mischflügel wird das Mischgut in dem Mischraum durch die Drehung der Mischflügel zusätzlich vorangetrieben und schließlich aus einer Austragsöffnung herausgetrieben.

Bevorzugt sind senkrecht zur Förderrichtung des Mischguts feststehende Scherelemente nach Art von Klingen zwischen den Rotationsbereichen der Mischflügel angeordnet. Wenn die Mischflügel über eine zentrale Drehachse im Mischraum angetrieben werden, können die Scherelemente zusätzlich zur Lagerung und Positionierung der Drehachse im Mischraum verwendet werden. Eine zentrale Drehachse ist bevorzugt, da hierdurch verhindert wird, dass sich im axialen Zentrum des Mischraums nicht durchmischte oder schlecht durchmischte Teile des Mischguts bis zur Austragsöffnung gefördert und damit aus der Vorrichtung ausgetragen werden können.

Um die geringe Drehung (den geringen Drehwinkel) des Hebels beziehungsweise um den geringen linearen Hub des Stifts für eine starke Durchmischung durch die Mischflügel nutzbar machen zu können, wurde im Rahmen der vorliegenden Erfindung gefunden, dass ein Getriebe verwendet werden kann, um die geringe Bewegung des Hebels oder des Stifts zu einer höheren Umdrehungszahl und Winkelgeschwindigkeit der Mischflügel umzusetzen.

Dennoch kann und soll erfindungsgemäß ein einziger Hub des Hebels oder des Stifts nicht ausreichen, um die Kartuschen vollständig zu entleeren. Deshalb muss bei der Rückstellung des Hebels oder des Stifts, die erfindungsgemäß bevorzugt mit einem Rückstellelement wie einer Feder erreicht wird, verhindert werden, dass die Förderkolben wieder zurückgezogen werden und dass sich die Mischflügel in umgekehrtem Drehsinn drehen und damit das Mischgut zurück in Richtung der Kartuschen drücken. Für die gerichtete Drehung der Mischflügel wird erfindungsgemäß eine Freilaufkupplung oder eine andere Vorrichtung zum Erreichen oder Erzwingen einer Vorzugsrichtung der Drehung verwendet, die in der Verbindung zwischen dem Hebel oder dem Stift und den Mischflügeln angeordnet ist. Zur Verhinderung einer Rückwärtsbewegung der Förderkolben oder der Einrichtung zum Antreiben der Förderkolben wird eine Zahnstange mit einer einseitig wirkenden Rastung verwendet, wie diese beispielsweise in einem Wagenheber vorkommen, oder es wird eine Klemmvorrichtung verwendet, mit der eine Klemmstange durch Verkippen der Klemmvorrichtung eingeklemmt und in einer Richtung vorgetrieben wird, während sich die Klemmvorrichtung bei einer umgekehrten Bewegung von der Stange löst und damit die Stange nicht in die umgekehrte Richtung bewegen kann. Solche Klemmvorrichtungen sind bei Kartuschenpistolen wie beispielsweise Kartuschenskelettpistolen bekannt.

Durch den gleichzeitigen Antrieb der Ausgangskomponenten in und aus den Kartuschen und des Mischguts im Mischraum über ein Getriebe können auch sehr zähe und hochviskose pastenförmige Zemente und Zementkomponenten noch manuell ausgepresst und gemischt werden. Die Energie zum Mischen der Ausgangskomponenten muss mit der erfindungsgemäßen Vorrichtung und dem erfindungsgemäßen Verfahren nicht mehr aus der Kraft des Vortriebs der Ausgangskomponenten aus den Kartuschen entnommen werden. Stattdessen wird das Mischgut selbst durch die aktive Bewegung der Mischflügel gemischt und bevorzugt durch eine geeignete Stellung der Mischflügel vorgetrieben. Die Kraft für die Durchmischung muss deshalb nicht mehr aus dem hydrostatischen Druck der Ausgangskomponenten entnommen werden sondern wird unmittelbar im Mischraum von den Mischflügeln auf das Mischgut übertragen.

Durch das Getriebe kann der geringe Hub des Hebels oder des Stifts verwendet werden, um eine starke Durchmischung und einen starken Vortrieb durch eine verhältnismäßig schnelle Umdrehung der Mischflügel zu ermöglichen.

Eine bevorzugte erfindungsgemäße, manuell bedienbare Vorrichtung ist zum Lagern und Mischen von Ausgangskomponenten eines medizinischen Zements, insbesondere eines PMMA-Knochenzements, geeignet und ist ferner zum Applizieren des gemischten medizinischen Zements geeignet und kann beispielsweise aufgebaut sein mit mehreren Kartuschen (bevorzugt zwei Kartuschen) mit zylindrischem Innenraum, wobei in jeder Kartusche ein zylindrischer, den Innenraum der Kartusche abschießender und axial beweglicher zylindrischer Förderkolben angeordnet ist, mit dem die Ausgangskomponenten aus dem Innenraum der Kartusche ausgetrieben werden können. Die Ausgangskomponenten werden dabei durch eine den Förderkolben gegenüberliegenden Öffnung in einen Mischraum geleitet. Dort werden die Ausgangskomponenten durch sich drehende Mischflügel durchmischt und vorgetrieben. Schließlich wird das durchmischte Mischgut vorne aus einer Austragsöffnung oder bevorzugt durch ein Applikationsrohr oder Austragsrohr ausgetrieben und appliziert. Zur Bedienung der Vorrichtung wird ein Hebel verwendet, der im Bereich eines Pistolengriffs angeordnet ist. Die Vorrichtung ist in einem bis auf die Austragsöffnung oder den Anschluss für das Austragsrohr und bis auf eine Durchführung für den Hebel geschlossenes Gehäuse angeordnet. Der Hebel ist mit einer Klemmvorrichtung zum unidirektionalen Vortreiben einer Stange verbunden. Die Stange ist mit einem Joch verbunden, mit der die Förderkolben in den Kartuschen vorangetrieben werden. Das Joch ist entweder lang genug, um auch bei vollständig in Richtung der Öffnungen zum Mischraum vorgetriebenen Förderkolben noch um die rückseitigen Wände der Kartuschen herum zu reichen, oder das Joch weist Schneidkanten auf, die sich beim Vortreiben der Förderkolben durch die Wände der Kartusche schneiden. Die Klemmvorrichtung greift in die Stange, wenn der Hebel manuell durch zusammendrücken mit der Hand, die auch den Pistolengriff hält, zum Pistolengriff gezogen wird, und bewegt die Stange mit dem Joch und damit die Förderkolben in die Kartuschen hinein, wodurch die Ausgangskomponenten in dem Mischraum gedrückt werden. Wenn der Hebel von einem Rückstellelement, das beim Drücken des Hebels gespannt wurde, wieder in die Ausgangsstellung rücküberführt wird, löst sich die Klemmvorrichtung und wird gegen die Stange bewegt, so dass sich die Stange und das Joch nicht wieder zurück bewegt und von den Förderkolben lösen kann.

Mit der gleichen Bewegung des Hebels wird auch ein Zahnradkranz gedreht, der konzentrisch um den Drehpunkt des Hebels angeordnet ist. In die Zähne des Zahnradkranzes greift ein erstes kleines Zahnrad, dessen Drehachse mit einem größeren Zahnrad mit einem größeren Durchmesser und mehr Zähnen als das kleine Zahnrad verbunden ist. Die Zahnräder können durch Kegelzahnräder oder Kronenräder realisiert werden, um die Drehrichtung der Antriebswelle der Mischflügel gegenüber der Drehrichtung des Hebels zu ändern. In der Antriebswelle, die die Drehachse der Mischflügel bildet, ist eine Freilaufkupplung beziehungsweise eine gefederte Drehkupplung angeordnet. Auf der den Mischflügeln zugewandten Seite der Drehachse wird sich diese dann nur noch in einer Richtung drehen lassen, so dass die Mischflügel im Mischraum nur in einer Richtung gedreht werden können. Die Mischflügel sind dabei derart gegen die Drehachse in dem Mischraum geneigt, dass das Mischgut im Mischraum in Richtung der Austragsöffnung getrieben wird.

Alternativ zu der Ausgestaltung mit Zahnrädern können auch Reibräder verwendet werden.

Die bei erfindungsgemäßen Vorrichtungen und Verfahren bevorzugt als Mischgut verwendeten Polymethylmethacrylat-Knochenzemente verwenden besonders bevorzugt Methylmethacrylat als Monomer und damit als eine erste Ausgangskomponente ein Pulver oder eine Paste enthaltend Methylmethacrylat. Dazu passende Redoxinitiatorsysteme bestehen üblicherweise aus Peroxiden, Beschleunigern sowie gegebenenfalls geeigneten Reduktionsmitteln, die dann erfindungsgemäß in der zweiten Ausgangskomponente enthalten sind. Eine Radikalbildung erfolgt nur dann, wenn alle Bestandteile der Redoxinitiatorsysteme zusammenwirken. Deshalb werden die Ausgangskomponenten des Redoxinitiatorsystems in den durch die räumlich getrennten Kartuschen separierten Zementpasten so angeordnet, dass diese keine radikalische Polymerisation auslösen können. Die Trennung wird durch das Ventilelement oder die Verschlüsse oder eine andere zu öffnende Trennvorrichtung erreicht, mit der die Ausgangskomponenten zuverlässig getrennt werden können. Die Ausgangskomponenten sind so bei geeigneter Zusammensetzung und geeigneter Wahl des Materials der Kartuschen lagerstabil. Erst bei Vermischung der Ausgangskomponenten zu einem Zementteig als Mischgut reagieren die zuvor getrennt in beiden gelagerten Bestandteile des Redoxinitiatorsystems, wobei Radikale gebildet werden, welche die radikalische Polymerisation des wenigstens einen Monomers auslösen. Die radikalische Polymerisation führt dann unter Verbrauch des Monomers zu Bildung von Polymeren, wobei der Zementteig (insbesondere nach der Applikation des Zementteigs) aushärtet. Zur Vermischung der Ausgangskomponenten und zum Vortrieb des Zementteigs wird ein dynamischer Mischer, das heißt, die sich im Mischraum drehenden Mischflügel eingesetzt.

Im Folgenden werden weitere Ausführungsbeispiele der Erfindung anhand von fünf schematisch dargestellten Figuren erläutert, ohne jedoch dabei die Erfindung zu beschränken. Dabei zeigt:
- Figur 1:: eine schematische perspektivische Ansicht einer aufgeklappten erfindungsgemäßen Vorrichtung;
- Figur 2:: eine schematische perspektivische Teilansicht einer erfindungsgemäßen Vorrichtung;
- Figur 3:: eine schematische perspektivische teilweise geschnittene Ansicht einer erfindungsgemäßen Vorrichtung;
- Figur 4:: eine weitere schematische perspektivische teilweise geschnittene Ansicht einer erfindungsgemäßen Vorrichtung; und
- Figur 5:: eine schematische Seitenansicht eines Teils einer weiteren erfindungsgemäßen Vorrichtung.

In den Figuren werden teilweise für gleiche oder gleichartige Bauteile die gleichen Bezugszeichen verwendet, auch wenn es sich um unterschiedliche Vorrichtungen beziehungsweise Zementierpistolen handelt.

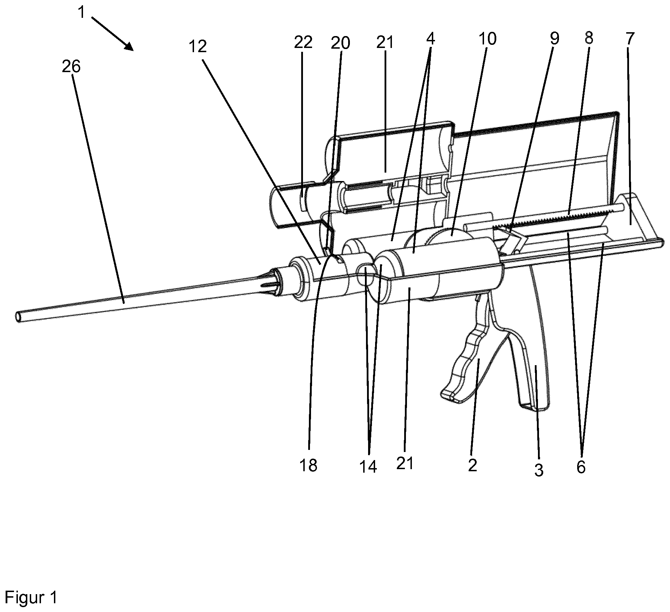

Figur 1 zeigt eine schematische perspektivische Ansicht einer nach oben geöffneten erfindungsgemäßen Vorrichtung 1. Die Vorrichtung 1 weist einen manuell ziehbaren Hebel 2 auf, der vor einem Pistolengriff 3 angeordnet ist. Die Vorrichtung 1 ist dadurch mit einer Hand zu halten und der Hebel 2 ist mit der gleichen Hand zu bedienen, mit der auch der Hebel 2 gezogen beziehungsweise bedient werden kann. Der Hebel 2 ist drehbar um eine Achse gegen den Griff 3 gelagert, wobei eine elastische Druckfeder (nicht gezeigt) zwischen dem Hebel 2 und dem Griff 3 angeordnet ist, so dass der Hebel 2 mit der Druckfeder immer in die gezeigte Ausgangsposition zurück überführt wird. Der Hebel 2 weist drei Mulden zur Auflage von Fingern auf, so dass er bequem mit den Fingern der Hand bedient werden kann, die auch den Griff 3 hält.

Im Inneren der Vorrichtung 1 befinden sich zwei nebeneinander angeordnete zylindrische Kartuschen 4, in denen sich die Ausgangskomponenten (nicht gezeigt) befinden, die zur Herstellung des Mischguts verwendet werden. Zudem sind Rückseitig (in Figur 1 rechts) in den Kartuschen 4 in Längsrichtung verschiebbare Förderkolben (in Figur 1 nicht zu sehen) angeordnet. Die Förderkolben sind auf ihrer Rückseite (in Figur 1 rechts) mit zwei Stangen 6 verbunden oder die Stangen 6 liegen rückseitig an den Förderkolben an. Die Stangen 6 sind über ein Joch 7 miteinander verbunden. Die Stangen 6 dienen dazu, dass sie die Förderkolben in den Kartuschen 4 bis zum vorderen Ende der Kartuschen 4 (in Figur 1 links) drücken können, ohne dass das Joch an der rückseitigen Öffnung der Kartuschen 4 anliegt und dadurch eine weitere Bewegung der Stangen 6 ins Innere der Kartuschen 4 und damit eine weitere Bewegung der Förderkolben in Richtung der Vorderseite der Kartuschen 4 (in Figur 1 links) verhindert.

Das Joch 7 ist zudem mit einer Zahnstange 8 verbunden, wobei die Zähne der Zahnstange 8 eine steile Flanke (in Richtung der Vorderseite - in Figur 1 links) und eine flache Flanke (in Richtung der Rückseite - in Figur 1 rechts) aufweisen. Alternativ kann das Joch 7 auch durch eine Klinge gebildet werden, die die Stangen 6 mit der Zahnstange 8 verbinden und die sich durch die Wandungen der Kartuschen 4 schneiden, wenn das Joch 7 und die Förderkolben nach vorne getrieben werden. Dieser Aufbau hat den Vorteil, dass die Vorrichtung 1 kompakter aufgebaut werden kann, da die Stangen 6 dann wesentlich kürzer gestaltet werden können, wobei die Zahnstange 8 dann in ihrer Ausgangsposition weiter in Richtung der Vorderseite der Vorrichtung 1 angeordnet ist, hat aber auch den Nachteil, dass eine größere mechanische Kraft aufgewendet werden muss, um die Klingen des Jochs durch die Wände der Kartuschen 4 schneiden zu lassen. Zudem können solche Klingen nicht aus Kunststoff gefertigt werden. Daher ist der gezeigte Aufbau mit dem breiten Joch 7 bevorzugt.

In die Zähne der Zahnstange 8 greift eine Rastung 9, die über eine Achse beweglich mit dem Hebel 2 verbunden ist und die elastisch gefedert gegen die Zähne der Zahnstange 8 gelagert ist. Die Rastung 9 greift mit ihrer vorderen Kante in die steilen Flanken der Zähne der Zahnstange 8, wenn der Hebel 2 durch Bewegen des Hebels in Richtung des Griffs 3 gedreht beziehungsweise gekippt wird, da die Achse, um die der Hebel 2 gedreht wird, unterhalb der Verbindung zur Rastung 9 liegt. Der obere Teil des Hebels 2 kippt also in die entgegengesetzte Richtung zum unteren Teil des Hebels 2, an dem dieser gegriffen wird.

Dadurch wird die Zahnstange 8 nach vorne (in Figur 1 nach links) getrieben und mit der Zahnstange 8 das Joch 7 und die Stangen 6 und dadurch die Förderkolben in den Kartuschen 4 nach vorne getrieben, so dass die Ausgangskomponenten aus den Kartuschen 4 nach vorne ausgetrieben werden. Wenn der Hebel 2 losgelassen wird oder kein Druck mehr oder ein ausreichend geringer Druck mit der Hand des Anwenders auf den Hebel 2 ausgeübt wird, sorgt die Federung des Hebels 2 dafür, dass sich die Rastung 9 in Richtung der Rückseite der Vorrichtung 1 bewegt. Dabei wird die Rastung 9 gegen den Hebel 2 gekippt und rutscht über die flachen Flanken der Zähne der Zahnstange 8, so dass die Zahnstange 8 nicht in Richtung der Rückseite der Vorrichtung 1 (in Figur 1 rechts) bewegt wird.

Durch die Federung der Rastung 9 greift diese beim nächsten Drücken beziehungsweise beim nächsten Ziehen des Hebels 2 zum Griff 3 wieder in weiter hinten (in Figur 1 weiter rechts) angeordnete Zähne der Zahnstange 8 ein. Dadurch kann die Zahnstange 8 und damit die Stangen 6 und die Förderkolben mit jedem Hub des Hebels 2 ein paar Zähne beziehungsweise Zahnlängen weiter in Richtung der Vorderseite der Vorrichtung 1 gedrückt werden.

Der Hebel 2 ist zusätzlich mit einem Zahnkranz 10 verbunden, der mit dem Drehen des Hebels 2 ebenfalls gedreht wird. An einer Seitenfläche des Zahnkranzes 10 sind Zähne angeordnet, die in die Zahnräder eines Getriebes (in Figur 1 nicht zu sehen) greifen. Über dieses Getriebe werden Mischflügel (in Figur 1 nicht zu sehen) angetrieben, die sich auf einer Drehachse (in Figur 1 nicht zu sehen) in einem Mischraum 12 drehen können. Der Mischraum 12 ist über eine Verbindung 14 mit dem Innenraum der Kartuschen 4 verbunden, so dass beim Vortreiben der Förderkolben die Ausgangskomponenten aus den Kartuschen 4 über die Verbindung 14 in den Mischraum 12 geleitet wird.

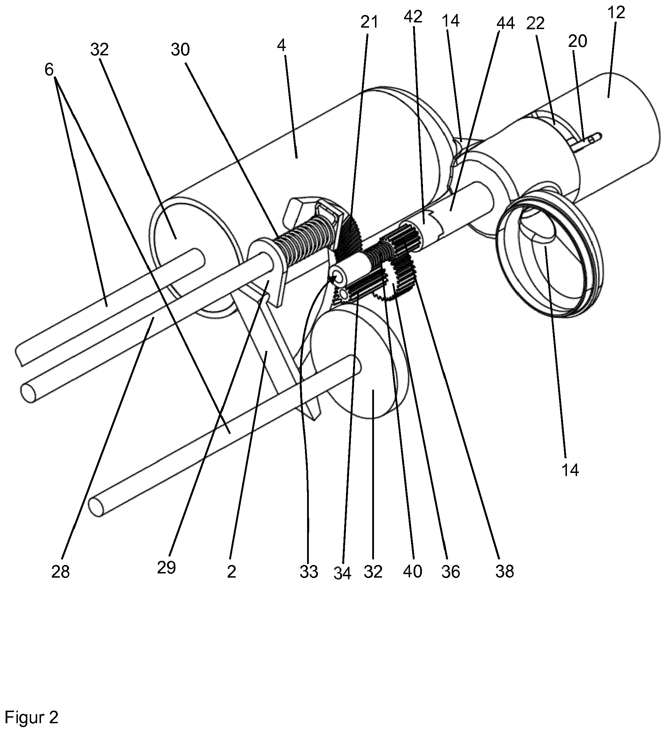

In dem Mischraum 12 werden die Ausgangskomponenten durch Drehung der Mischflügel gemischt sowie in Richtung eines Austragsrohrs 16 an der Vorderseite der Vorrichtung 1 gefördert und schließlich durch das Austragsrohr 16 ausgetrieben. Mit Hilfe einer Freilaufkupplung (in Figur 1 nicht gezeigt) wird erreicht, dass die Mischflügel sich nur in einer Richtung beziehungsweise in einem Drehsinn in dem Mischraum 12 drehen können. Bei der zur Ausgangsposition gerichteten Bewegung des Hebels 2, die durch die Druckfeder zwischen dem Hebel 2 und dem Griff 3 erreicht wird, drehen sich die Mischflügel also nicht mit. Durch eine geeignete Übersetzung des Getriebes wird erreicht, dass sich die Mischflügel bei einem vollen Hub des Hebels 2 zweimal bis fünfmal um die Drehachse drehen. Beispielhafte Aufbauten des Getriebes und der Vorrichtung für den Freilauf, beziehungsweise der Freilaufkupplung sind in den Figuren 2 bis 4 beschrieben, wo auch deren Funktionsweise genauer erläutert wird.

Die Kartuschen 4 sind zunächst mit einem Ventilelement 18 verschlossen, das drehbar als Rohrstück in dem der Verbindung 14 zugewandten Teil der Mischraums 12 angeordnet ist. Das Ventilelement 18 weist zwei Öffnungen (nicht zu sehen) auf, die in Überdeckung mit den Öffnungen der Verbindung 14 gebracht werden können. Hierzu kann das Ventilelement 18 über einen Ventilhebel 20 gedreht werden. Der Ventilhebel 20 erstreckt sich durch einen Spalt in der Wandung des Mischraums 12.

Der gesamte Aufbau ist in einem Gehäuse 21 angeordnet und aus Kunststoff gefertigt. Die Federelemente und Druckfedern können auch aus Metall gefertigt sein.

Ein oberer Teil des Gehäuses 21 ist in Figur 1 nach oben aufgeklappt dargestellt, um einen Blick ins Innere der Vorrichtung 1 zu ermöglichen. Der Ventilhebel 20 des Ventilelements 18 ragt durch einen Schlitz 22 in dem Gehäuse 21. Im Betrieb ist das Gehäuse 21 geschlossen und damit alle beweglichen Teile abgedeckt. Zum Bereitmachen der Vorrichtung 1 beziehungsweise der Zementierpistole 1 wird zunächst der Ventilhebel 20 gedreht und damit die Kartuschen 4 zum Mischraum 12 geöffnet. Anschließend wird der Hebel 2 wiederholt bedient (gedrückt) und dadurch die Ausgangskomponenten des Zements in den Kartuschen 4 in den Mischraum 12 gepresst und dort gemischt. Mit Hilfe des Drucks aus den Kartuschen 4 und mit Hilfe des Antriebs durch die Mischflügel im Mischraum 12 wird das Zementgemisch durchmischt und nach vorne durch das Austragsrohr 16 ausgetrieben und kann dort appliziert werden.

Figur 2 zeigt eine schematische perspektivische Teilansicht einer erfindungsgemäßen Vorrichtung. Die Vorrichtung ist in Figur 2 ohne ihr Gehäuse dargestellt und der Ausschnitt konzentriert sich auf die wesentlichen Teile des Antriebs der Vorrichtung. Die Vorrichtung weist zwei parallel zueinander angeordnete Kartuschen 4 auf, von denen in Figur 2 nur eine eingezeichnet ist. Die zweite Kartusche wurde nicht eingezeichnet, um einen besseren Blick auf das Getriebe zu ermöglichen. Die Vorrichtung ähnelt der nach Figur 1, wobei bei dem vorliegenden Aufbau statt der Zahnstange eine einfache glatte zylindrische Klemmstange 28 verwendet wird, in die anstatt der Rastung eine kippbare Verkantungsscheibe 29 mit einer Verkantungsöffnung greift. Die Verkantungsscheibe 29 weist also eine zentral angeordnete Verkantungsöffnung auf, durch die sich die Klemmstange 28 erstreckt, wobei der Innendurchmesser der Verkantungsöffnung größer ist als der Außendurchmesser der Klemmstange 28, so dass die Verkantungsscheibe 29 auf der Klemmstange 28 geneigt werden kann und dadurch die Verkantungsöffnung mit der Klemmstange 28 verkanten kann. Solche Klemmstangen 28 sind bei Zementierpistolen und Zementierskelettpistolen bekannt.

Die Verkantungsscheibe 29 ist um eine Achse kippbar beziehungsweise drehbar mit einem Hebel 2 verbunden, der auf der Unterseite (nicht gezeigt) manuell gedreht werden kann. Durch eine Feder 30 wird die Verkantungsscheibe 29 in eine rutschfähige (nicht verkantete) Position überführt, wenn der Hebel 2 nicht bedient wird. Wenn der Hebel 2 bewegt wird, wenn also der in Figur 2 zu sehende Teil des Hebels 2 nach vorne (in Figur 2 nach rechts oben) bewegt wird, verkantet die Verkantungsscheibe 29 mit der Klemmstange 28. Dadurch wird die Klemmstange 28 nach vorne (in Figur 2 nach rechts oben) gedrückt.

Mit der Klemmstange 28 sind zwei Stangen 6 über ein Joch (nicht gezeigt) verbunden, von denen nur eine Stange 6 in Figur 2 dargestellt ist. Wenn die Klemmstange 28 also nach vorne bewegt wird, bewegt sich auch die Stange 6 nach vorne und drückt zwei Förderkolben 32 in die zwei parallel angeordneten Kartuschen 4, wodurch der Inhalt der Kartuschen 4 nach vorne aus den Kartuschen 4 in eine Verbindung 14 und von dort in einen Mischraum 12 gepresst wird.

An dem Hebel 2 ist ein Zahnkranz 10 befestigt, der sich dreht, wenn der Hebel 2 gedreht wird. In dem Mischraum 12 ist eine drehbar gelagerte Drehachse 33 angeordnet, an der Mischflügel (in Figur 2 nicht zu sehen) angeordnet sind, die sich im Mischraum 12 um die Drehachse 33 drehen können. Die Drehachse 33 ist von dem Zahnkranz 10 über ein Getriebe antreibbar. In den Zahnkranz 10 greift ein erstes kleines Zahnrad 34 des Getriebes, das über eine Achse mit einem zweiten größeren Zahnrad 36 des Getriebes verbunden ist. Das zweite größere Zahnrad 36 greift schließlich in ein drittes kleines Zahnrad 38, das auf der Drehachse 33 befestigt ist. Durch diese Übersetzung wird erreicht, dass sich die Drehachse 33 bei einem vollen Hub des Hebels 2 und damit bei einer ca. 45° Drehung des Zahnkranzes 10 etwa drei mal um die eigene Achse dreht. Die Mischflügel drehen sich in dem Mischraum 12 entsprechend.

Zwischen dem dritten Zahnrad 38 und dem Mischraum 12 ist in der Drehachse 33 eine Freilaufkupplung beziehungsweise eine gefederte Drehkupplung angeordnet. Die Federung 40 hierfür ist auf der Drehachse 33 erkennbar. Die Freilaufkupplung beziehungsweise die gefederte Drehkupplung wird durch zwei ineinander greifende verzahnte Kreisscheiben 42, 44 realisiert, die durch die Federung 40 aufeinander gedrückt werden. Durch bezogen auf den Drehsinn unterschiedlich steile Flanken der Zähne der Kreisscheiben 42, 44 wird eine Vorzugsrichtung der Drehung des Teils der Drehachse 33 erreicht, der sich ins Innere des Mischraums 12 fortsetzt.

Die Öffnungen der Verbindung 14 zum Mischraum 12 kann durch ein Ventilelement analog dem nach Figur 1 verschlossen und geöffnet werden. Das Ventilelement kann über einen Ventilhebel 20 in einem Schlitz 22 gedreht und dadurch geöffnet oder geschlossen werden. Die Funktionsweise der Vorrichtung nach Figur 2 gleicht der Funktionsweise der Vorrichtung 1 nach Figur 1. Bei jedem Hub des Hebels 2 werden die Förderkolben 32 weiter ins Innere der Kartuschen 4 vorgetrieben. Gleichzeitig wird mit der gleichen Bewegung des Hebels 2 die Drehachse 33 gedreht und damit die Mischflügel in dem Mischraum 12 gedreht. Durch die Drehung der Mischflügel wird der Inhalt des Mischraums 12 durchmischt und durch eine Austrittsöffnung (in Figur 2 nicht zu sehen) aus der Vorrichtung ausgepresst.

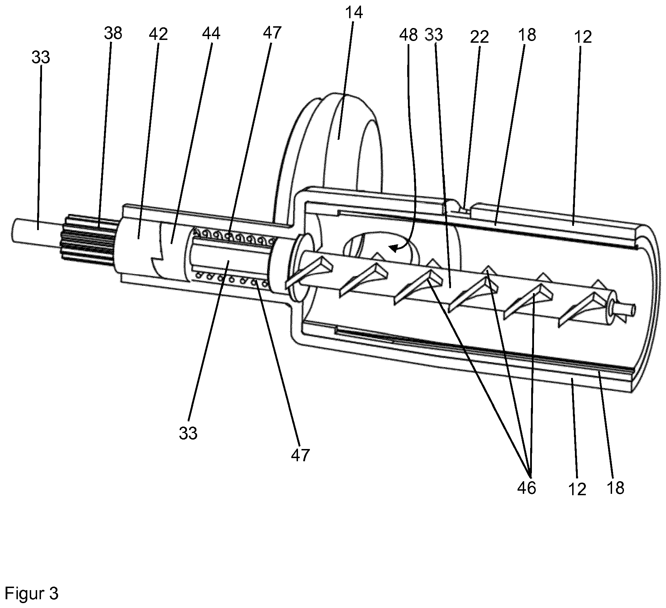

Figur 3 zeigt eine schematische perspektivische und teilweise geschnittene Ansicht der erfindungsgemäßen Vorrichtung nach Figur 2, in der die beiden ineinander greifenden und verzahnten Kreisscheiben 42, 44 der Freilaufkupplung beziehungsweise der gefederten Drehkupplung und die Mischflügel 46 gut zu erkennen sind. Zudem ist durch den Schnitt zu erkennen, wie das Ventilelement 18 in den Wänden des Mischraums 12 sitzt beziehungsweise anliegt. Eine Feder 47 drückt die rechte (vordere) Kreisscheibe 44 auf die linke (hintere) Kreisscheibe 42. Auf der Drehachse 33 sind die Mischflügel 46 gleichsinnig gegen die Drehachse 33 geneigt angeordnet, so dass bei einer Drehung der Drehachse 33 ein Vortrieb des Mischguts im Mischraum 12 erfolgt (in Figur 3 nach rechts).

Wenn sich die obere Kante des (dritten) Zahnrads 38 in die Bildebene hinein dreht, reicht die Reibung der flachen Flanken der Kreisscheiben 42, 44 nicht aus, damit sich die rechte Kreisscheibe 44 mit der linken Kreisscheibe 42 dreht. Die Kreisscheiben 42, 44 rutschen übereinander und die im Mischraum 12 angeordnete Drehachse 33 dreht sich nicht.

Wenn sich die untere Kante des (dritten) Zahnrads 38 in die Bildebene hinein dreht, greifen die steilen (senkrechten) Flanken der Kreisscheiben 42, 44 ineinander, so dass sich die rechte Kreisscheibe 44 mit der linken Kreisscheibe 42 dreht. Die im Mischraum 12 angeordnete Drehachse 33 dreht sich mit den Mischflügeln 46. Bei einer wiederholten Bewegung des Hebels 2 und damit der über das Getriebe vermittelten wiederholten Drehbewegung der Drehachse 33 vor dem Mischraum 12 erfolgt also nur eine unidirektionale Drehung der Drehachse 33 im Mischraum 12 und damit der Mischflügel 46. Die Mischflügel 46 treiben das Mischgut im Mischraum 12 also immer zur Austragsöffnung (in Figur 3 rechts).

Das Ventilelement 18 hat eine runde Ventilöffnung 48 in Form eines Durchgangs durch die Wandung des rohrförmigen Ventilelements 18. Die Ventilöffnung 48 dreht sich mit dem Ventilelement 18. Wenn das Ventilelement 18 in eine geeignete Position gedreht wird, wie in Figur 3 gezeigt, überdeckt die Ventilöffnung 48 den Zugang zur Verbindung 14 beziehungsweise zum Kartuschenanschluss 14. Eine zweite Ventilöffnung (nicht zu sehen) stellt dann eine Öffnung zur Verbindung 14 der zweiten Kartusche (in Figur 3 nicht zu sehen) her. Dadurch sind die beiden Kartuschen in den Mischraum 12 geöffnet und die Ausgangskomponenten können aus den Kartuschen in den Mischraum 12 gefördert werden.

Gemäß einer weiteren Ausführungsform der Erfindung, die als Abwandlung des Aufbaus nach Figur 3 angesehen werden kann, kann vorgesehen sein, dass das dritte Zahnrad 38 und die linke Kreisscheibe 42 eine zylindrische Durchführung aufweisen, und die Drehachse 33 sich durch diese Durchführungen hindurch erstreckt, so dass das dritte Zahnrad 38 und die linke Kreisscheibe 42 entlang der Symmetrieachse der Drehachse 33 verschiebbar auf der Drehachse 33 angeordnet sind. Die Drehachse 33 ist dann in Richtung des Jochs als Spindel mit einem Gewinde ausgeformt. In dem Joch, mit dem die Stangen und damit die Förderkolben vorangetrieben werden, ist ein Innengewinde nach Art einer Mutter vorgesehen, so dass das Joch und damit die Stangen und die Förderkolben durch die Drehung der Spindel in Richtung der Verbindungen 14 der Kartuschen zum Mischraum 12 gezogen werden. Das dritte Zahnrad 38 und die linke Kreisscheibe 42 sind zudem durch eine Druckfeder zwischen dem Gewinde der Spindel und dem dritten Zahnrad 38 federnd in Richtung der rechten Kreisscheibe 44 gelagert. Das dritte Zahnrad 38 und die linke Kreisscheibe 42 sind bevorzugt einteilig ausgeführt. Das dritte Zahnrad 38 hat dazu bevorzugt eine größere Höhe als das in das dritte Zahnrad 38 eingreifende Antriebszahnrad (das zweite Zahnrad), so dass das dritte Zahnrad 38 auf dem Antriebszahnrad gleiten kann, ohne dass die volle Höhe des Antriebszahnrads nicht mehr nutzbar wäre. Dazu reicht es aus, wenn das dritte Zahnrad 38 um die Höhe der Zähne der Kreisscheiben 42, 44 höher ist, als das Antriebszahnrad. Durch diesen Aufbau wird erreicht, dass die sich drehende Drehachse 33 der Mischflügel 46 auch als Antriebsspindel für den Vortrieb der Förderkolben verwendet werden kann.

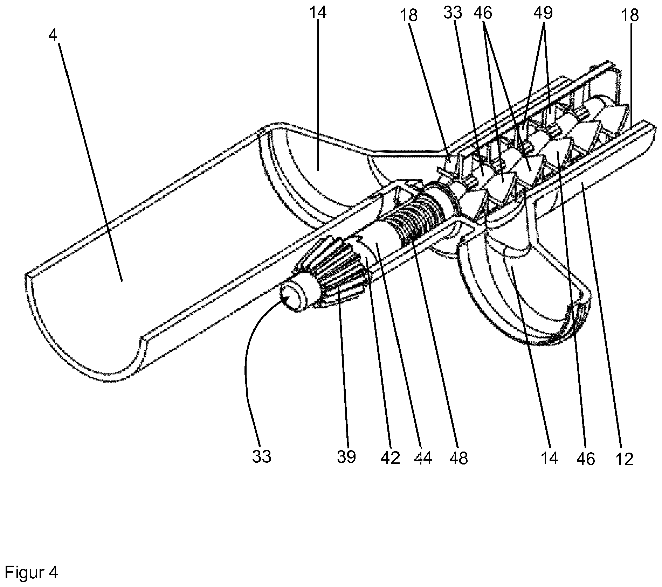

Figur 4 zeigt eine weitere schematische perspektivische und teilweise geschnittene Ansicht einer erfindungsgemäßen Vorrichtung. Der Aufbau gleicht dem nach den Figuren 2 und 3, wobei ein Kegelzahnrad 39 anstatt der normalen Zahnräder eingebaut ist, so dass eine Umlenkung des Drehsinns durch das Getriebe erfolgen kann. Die Zähne des Zahnkranzes (in Figur 4 nicht gezeigt) können dadurch auch auf der Stirnseite des Zahnkranzes angeordnet sein. Dazu sind dann auch die anderen Zahnräder teilweise oder alle als Kegelzahnräder (nicht gezeigt) ausgebildet. Bevorzugt weist auch der Zahnkranz eine geneigte Oberfläche auf und bildet dadurch einen Kegelzahnkranz.

Im Mischraum 12 sind statische Scherelemente 49 angeordnet, die sich nicht mit der Drehachse 33 drehen. Diese Scherelemente 49 dienen dazu, das nach vorne (in Figur 4 nach rechts oben hinten) getriebene Mischgut senkrecht zur Förderrichtung zu schneiden und dadurch die Durchmischung zu verbessern. Von der gegenüberliegenden Seite der Drehachse 33 ist im Mischraum 12 eine spiegelbildlich angeordnete Reihe von fünf Scherelementen angeordnet, die in Figur 4 nicht zu sehen sind. Dadurch wird die Drehachse 33 durch die Scherelemente 49, die in Zylinderschalensegmenten enden, im Mischraum 12 gelagert.

Die Federung 47 dient dazu, die rechte (vordere) Kreisscheibe 44 auf die linke (hintere) Kreisscheibe 42 zu drücken. Wenn sich die obere Kante des Kegelzahnrads 39 bezogen auf die Drehachse 33 (von hinten aus betrachtet - in Figur 4 von links unten vorne aus betrachtet) gegen den Uhrzeigersinn dreht, reicht die Reibung der flachen Flanken der Kreisscheiben 42, 44 nicht aus, damit sich die rechte Kreisscheibe 44 mit der linken Kreisscheibe 42 dreht. Die Kreisscheiben 42, 44 rutschen stattdessen übereinander und der im Mischraum 12 angeordnete Teil der Drehachse 33 und die Mischflügel 46 drehen sich nicht.

Wenn das Kegelzahnrad 39 mit dem Uhrzeigersinn dreht (in Figur 4 von links unten vorne aus betrachtet), greifen die steilen (senkrechten) Flanken der Kreisscheiben 42, 44 ineinander, so dass sich die rechte Kreisscheibe 44 mit der linken Kreisscheibe 42 dreht. Die im Mischraum 12 angeordnete Drehachse 33 dreht sich mit den Mischflügeln 46. Bei der wiederholten Bewegung des Hebels 2 und damit der über das Getriebe vermittelten wiederholten Drehbewegung der Drehachse 33 vor dem Mischraum 12 erfolgt also nur eine unidirektionale Drehung der Drehachse 33 im Mischraum 12 und damit der Mischflügel 46. Die Mischflügel 46 treiben das Mischgut im Mischraum 12 also immer zur Austragsöffnung.

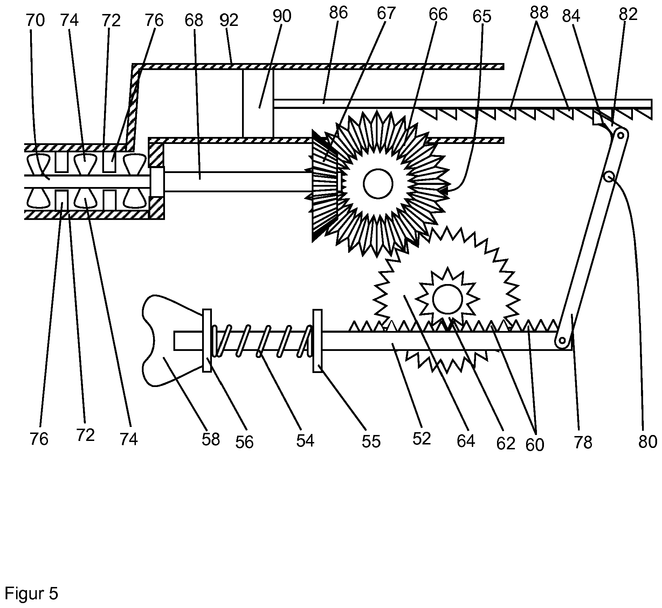

Figur 5 zeigt eine schematische Seitenansicht eines für die Funktion der Vorrichtung wesentlichen Teils einer weiteren erfindungsgemäßen Vorrichtung beziehungsweise einer erfindungsgemäßen Zementierpistole. Auch diese Vorrichtung ist ohne Gehäuse dargestellt. In der Abbildung nach Figur 5 ist ein Teil der Vorrichtung geschnitten dargestellt. Geschnittene Flächen sind in Figur 5 schraffiert dargestellt.

Die Vorrichtung wird anstelle eines Hebels mit einem in Längsrichtung beweglich und federnd gelagerten Stift 52 bedient. Die Federung wird mit Hilfe einer Feder 54 erreicht, die zwischen einer Halterung 55 am Gehäuse (nicht gezeigt) und einer Halterung 56 an einem Knopf 58 oder einer Taste 58 befestigt ist. Wenn der Knopf 58 in Richtung der Halterung 55 gedrückt wird, wird die Druckfeder 54 gestaucht und nimmt Energie auf. Beim Lösen des Drucks wird der Stift 52 mit dem Knopf 58 wieder nach vorne (in Figur 5 nach links) gedrückt.

Im hinteren Bereich, der im Inneren des Gehäuses angeordnet ist, sind auf dem Stift 52 auf einer Seite Zähne 60 angeordnet, so dass der Stift 52 in diesem Bereich eine Zahnstange 52 bildet. Diese Zahnstange 52 ist axial beweglich gegen ein Zahnrad 62 angeordnet, so dass die Zähne 60 der Zahnstange 52 beziehungsweise des Stifts 52 in die Zähne des Zahnrads 62 greifen. Das Zahnrad 62 ist über seine Achse mit einer Freilaufkupplung 64 verbunden, die in einem äußeren Zahnradkranz endet. Die Freilaufkupplung 64 dreht den äußeren Zahnradkranz nur dann mit dem Zahnrad 62, wenn das Zahnrad 62 sich bezogen auf die Blickrichtung des Betrachters der Figur 5 entgegen dem Uhrzeigersinn dreht, also wenn der Stift 52 nach hinten (in Figur 5 nach rechts) gedrückt wird. Solche Freilaufkupplungen 64 sind beispielsweise von Fahrrädern bekannt, können für den vorliegenden Zweck aber sehr einfach aufgebaut sein, da sie keine besonders leichtgängigen Leerlaufeigenschaften benötigen.

Der äußere Zahnkranz der Freilaufkupplung 64 greift in ein Zahnrad 65, das drehbar auf einer Zahnradachse angeordnet ist. Auf dieser Zahnradachse ist ein Kegelzahnrad 66 angeordnet, das sich mit dem Zahnrad 65 dreht. Das Kegelzahnrad 66 ist in Figur 5 vor dem Zahnrad 65 angeordnet, bezogen auf die Betrachtungsebene. In das Kegelzahnrad 66 greift ein zweites, orthogonal angeordnetes Kegelzahnrad 67, das eine Drehachse 68, 70 antreibt. Die Drehachsen der Kegelzahnräder 66, 67 sind also senkrecht zueinander. Die Neigung des Kegelmantels der Kegelzahnräder 66, 67 ist bezogen auf deren Drehachse in einem Winkel von 45° geneigt.

Das zweite Kegelzahnrad 67 ist auf der hinteren Drehachse 68 angeordnet. Die vordere Drehachse 70 ist in einem Mischraum 72 angeordnet, der durch Mischraumwände 72 begrenzt ist und der in Figur 5 als Querschnitt dargestellt ist. Die Drehachse 68, 70 ist dazu durch eine Dichtung in den Mischraum 72 geführt, in dem die Drehachse 68, 70 drehbar gelagert ist. Auf der vorderen Drehachse 70 sind Mischflügel 74 angeordnet, die sich beim Drehen der Drehachse 68, 70 im Mischraum 72 drehen. Die Mischflügel 74 sind gegen die Ebene senkrecht zur Drehachse 70 derart geneigt, dass die Mischflügel 74 ein Mischgut im Mischraum 72 nach vorne (in Figur 5 nach links) treiben, wenn die Drehachse 70 durch eindrücken des Stifts 52, beziehungsweise durch eine Bewegung des Stifts 52 in Figur 5 nach rechts, angetrieben wird.

Zur besseren Durchmischung des Mischguts sind im Mischraum 72 senkrechte Scherelemente 76 angeordnet, die sich als rechteckige Scheiben senkrecht von der Mischraumwand 72 in Richtung der Drehachse 70 erstrecken. Die Scherelemente 76 durchschneiden die in Richtung einer vorderen Austragsöffnung (in Figur 5 nicht gezeigt) getriebene Zementpaste (das Mischgut).

Der Stift 52 ist auf seiner Rückseite über eine drehbare Achse mit einem Hebel 78 verbunden. Der Hebel 78 ist über eine Achse 80 drehbar beziehungsweise kippbar gegen das Gehäuse gelagert. Der Hebel 78 kann teleskopartig aufgebaut sein, um die lineare Bewegung des Stifts 52 mitmachen zu können. Auf der gegenüberliegenden Seite des Hebels 78 ist ein Rastelement 82 über eine Achse drehbar mit dem Hebel 78 verbunden. Das Rastelement 82 wird mit einer Blattfeder 84 gegen eine Stange 86 mit Zähnen 88 gedrückt.

Die Zähne 88 der Stange 86 haben eine senkrechte Flanke, die in Figur 5 nach rechts weist, und eine flache Flanke, die in Figur 5 nach links weist. Das Rastelement 82 greift in die senkrechten Flanken der Zähne 88 und gleitet über die flachen Flanken der Zähne 88. Dadurch wird die Stange 86 bei einer Bewegung des Stifts 52 in Richtung der Rückseite der Vorrichtung (in Figur 5 nach rechts) durch den Hebel 78 und das Rastelement 82 in Richtung der Vorderseite der Vorrichtung (in Figur 5 nach links) gedrückt.

Die Stange 86 drückt auf einen Förderkolben 90, der in einer Kartusche 92 angeordnet ist, die mit einer der Ausgangskomponenten für das Mischgut, beziehungsweise mit dem Knochenzement gefüllt ist und die mit dem Mischraum 72 verbunden ist. Die Stange 86 kann auch an dem Förderkolben 90 befestigt sein. Genau vor oder auch hinter der in Figur 5 in Querschnittansicht gezeigten Kartusche 92 befindet sich eine zweite Kartusche (nicht zu sehen), die parallel zu der ersten Kartusche 92 angeordnet ist und in der ein Förderkolben (nicht zu sehen) linear 15160736.3 / P-HE1414EP 29 Heraeus Medical GmbH beweglich angeordnet ist. Der Aufbau der beiden parallelen Kartuschen 92 gleicht sich dabei. Die Stange 86 ist nach Art eines Jochs geformt, so dass auch der Förderkolben der vorderen (zweiten) Kartusche von der Stange 86 angetrieben wird. Die vordere Kartusche mündet ebenfalls in den Mischraum 72.

Durch den schubweise erfolgenden Vortrieb der Stange 86 und der Förderkolben 90 werden die Ausgangskomponenten aus den Kartuschen 92 in den Mischraum 72 gefördert und dort mit Hilfe der statischen Scherelemente 76 und der sich drehenden Mischflügel 74 durchmischt und mit Hilfe der sich drehenden Mischflügel 74 nach vorne getrieben. Schließlich wird das fertig gemischte Mischgut (der medizinische Zement) nach vorne aus der Vorrichtung ausgetrieben und appliziert.

An den Öffnungen oder in den Öffnungen zwischen den Kartuschen 92 und dem Mischraum 72 ist erfindungsgemäß bevorzugt zumindest ein manuell zu öffnender Verschluss (nicht gezeigt) oder ein manuell zu öffnendes Ventilelement (nicht gezeigt) angeordnet, mit dem der Mischraum 72 von den beiden Kartuschen 92 getrennt wird. Durch den Verschluss beziehungsweise das Ventilelement wird der Inhalt der Kartuschen 92 nach Außen abgeschlossen und die Vorrichtung ist somit zum Lagern der Ausgangskomponenten (beziehungsweise des Kartuscheninhalts) geeignet.

Anstelle von Zahnrädern und Kegelzahnrädern können bei allen Ausführungsformen auch Reibräder und Kegelreibräder verwendet werden.

### Bezugszeichenliste

- 1: Vorrichtung / Zementierpistole
- 2: Hebel
- 3: Griff / Pistolengriff
- 4: Kartusche
- 6: Stange
- 7: Joch
- 8: Zahnstange
- 9: Rastung
- 10: Zahnkranz
- 12: Mischraum
- 14: Verbindung / Kartuschenanschluss
- 16: Austragsrohr
- 18: Ventilelement
- 20: Ventilhebel
- 21: Gehäuse
- 22: Schlitz
- 28: Stange
- 29: Verkantungsstange
- 30, 47: Feder
- 32: Förderkolben
- 33: Drehachse
- 34, 36, 38: Zahnrad
- 39: Kegelzahnrad
- 40: Federung
- 42, 44: Kreisscheibe
- 46: Mischflügel
- 48: Ventilöffnung
- 49: Scherelement
- 52: Stift / Zahnstange
- 54: Feder
- 55, 56: Halterung
- 58: Knopf / Taste
- 60, 88: Zahn
- 62: Zahnrad
- 64: Freilaufkupplung
- 65: Zahnrad
- 66, 67: Kegelzahnrad
- 68: Hintere Drehachse
- 70: Vordere Drehachse
- 72: Mischraumwand
- 74: Mischflügel
- 76: Scherelement
- 78: Hebel
- 80: Achse
- 82: Rastelement
- 84: Blattfeder
- 86: Stange
- 90: Förderkolben
- 92: Kartusche

## Patentansprüche

1. Manuell antreibbare Vorrichtung (1) zum Mischen eines pastösen Mischguts aus wenigstens zwei fluiden Ausgangskomponenten, insbesondere zum Mischen eines medizinischen Zements, aufweisend
zumindest zwei Kartuschen (4, 92) enthaltend die fluiden Ausgangskomponenten oder zumindest zwei Anschlüsse für zumindest zwei Kartuschen (4, 92) enthaltend die fluiden Ausgangskomponenten, wobei in den Kartuschen (4, 92) Förderkolben (32, 90) zum Austreiben der Ausgangskomponenten aus den Kartuschen (4, 92) vorgesehen sind;
einen Mischraum (12, 72) zum Mischen der Ausgangskomponenten, der mit den Kartuschen (4, 92) oder den Anschlüssen für die Kartuschen (4, 92) verbunden ist, so dass beim Austreiben der Ausgangskomponenten aus den Kartuschen (4, 92) mit den Förderkolben (32, 90) die Ausgangskomponenten in den Mischraum (12, 72) geleitet werden, wobei in dem Mischraum (12, 72) Mischflügel (46, 74) vorgesehen sind, die in dem Mischraum (12, 72) drehbar gelagert sind;
einen um einen Drehpunkt drehbaren und manuell bedienbaren Hebel (2) zum Bedienen der Vorrichtung (1) oder einen in Längsrichtung verschiebbaren und manuell bedienbaren Stift (52) zum Bedienen der Vorrichtung (1);
ein Getriebe, das mit dem Hebel (2) oder dem Stift (52) verbunden ist, wobei das Getriebe mit den drehbaren Mischflügeln (46, 74) in dem Mischraum (12, 72) verbunden ist, so dass durch eine Bewegung des Hebels (2) oder des Stifts (52) die Mischflügel (46, 74) in dem Mischraum (12, 72) vermittelt über das Getriebe drehbar sind, und eine Einrichtung zum Vortreiben der Förderkolben (32, 90) in den Kartuschen (4, 92), die mit dem Hebel (2) oder dem Stift (52) verbunden ist, so dass bei einer Bewegung des Hebels (2) oder des Stifts (52) die Förderkolben (32, 90) in den Kartuschen (4, 92) vorzutreiben sind, so dass die Bewegung des Hebels (2) oder des Stifts (52) die Drehung der Mischflügel (46, 74) in dem Mischraum (12, 72) und die Bewegung der Förderkolben (32, 90) in die Kartuschen (4, 92) hinein antreibt,
**dadurch gekennzeichnet, dass**
in der Verbindung von dem Getriebe zu den Mischflügeln (46, 74) oder im Getriebe eine Kupplung (42, 44, 64) vorgesehen ist, die eine Drehung der Mischflügel (46, 74) in einer Drehrichtung ermöglicht und in der entgegengesetzten Drehrichtung verhindert oder reduziert.

2. Vorrichtung (1) nach Anspruch 1, **dadurch gekennzeichnet, dass**
die Mischflügel (46, 74) gegen die Achse, um die sich die Mischflügel (46, 74) im Mischraum (12, 72) drehen, im gleichen Sinn geneigt sind, so dass durch die Drehung der Mischflügel (46, 74) im Mischraum (12, 72) ein Vortrieb des Mischguts im Mischraum (12, 72) erfolgt, wobei bevorzugt die Mischflügel (46, 74) um den gleichen Winkel gegen die Achse geneigt sind.

3. Vorrichtung (1) nach Anspruch 1 oder 2, **dadurch gekennzeichnet, dass**
das Getriebe kraftschlüssig und/oder formschlüssig ist.

4. Vorrichtung (1) nach einem der vorangehenden Ansprüche, **dadurch gekennzeichnet, dass**
das Getriebe wenigstens eine Zahnscheibe, wenigstens eine Reibscheibe, wenigstens ein Zahnrad (10, 34, 36, 38, 39, 62, 65, 66, 67), wenigstens ein Schneckenrad und/oder wenigstens ein Reibrad aufweist, über das oder die die Kraft beziehungsweise das Drehmoment übertragen wird, wobei bevorzugt das Getriebe wenigstens eine Kegelzahnscheibe, wenigstens eine Kegelreibscheibe, wenigstens ein Kegelzahnrad (39, 66, 67) und/oder wenigstens ein Kegelreibrad zur Veränderung der Drehrichtung aufweist.

5. Vorrichtung (1) nach einem der vorangehenden Ansprüche, **dadurch gekennzeichnet, dass**
das Getriebe ein Zahnradgetriebe, ein Reibradgetriebe, ein Kegelradgetriebe, ein Kronenradgetriebe, ein Schneckengetriebe oder eine Kombination solcher Getriebe ist, bevorzugt zusätzlich aufweisend ein Stirnradgetriebe.

6. Vorrichtung (1) nach einem der vorangehenden Ansprüche, **dadurch gekennzeichnet, dass**
die Mischflügel (46, 74) auf einer drehbar gelagerten Drehachse (33, 68, 70) angeordnet sind, wobei die Drehachse (33, 68, 70) sich aus dem Mischraum (12, 72) heraus erstreckt, bevorzugt über eine Dichtung aus dem Mischraum (12, 72) heraus erstreckt, und die Drehachse (33, 68, 70) mit dem Getriebe verbunden ist, so dass durch eine Bewegung des Hebels (2) oder des Stifts (52) die Drehachse (33, 68, 70) mit den Mischflügeln (46, 74) in dem Mischraum (12, 72) drehbar ist.

7. Vorrichtung (1) nach einem der vorangehenden Ansprüche, **dadurch gekennzeichnet, dass**
die Kupplung (42, 44, 64) in der drehbar gelagerten Drehachse (33, 68, 70) vorgesehen ist.

8. Vorrichtung (1) nach einem der vorangehenden Ansprüche, **dadurch gekennzeichnet, dass**
an dem Getriebe oder in dem Getriebe eine Freilaufkupplung (64) vorgesehen ist oder in der Verbindung von dem Getriebe zu den Mischflügeln (46, 74), insbesondere in der drehbar gelagerten Drehachse (33, 68, 70), eine gefederte Drehkupplung (42, 44) vorgesehen ist.

9. Vorrichtung (1) nach einem der vorangehenden Ansprüche, **dadurch gekennzeichnet, dass**
die Einrichtung zum Vortreiben der Förderkolben (32, 90) in den Kartuschen (4, 92) eine Klemmstange (28) mit einer kippbaren Verkantungsöffnung oder eine Zahnstange (8, 86) mit einer unidirektional wirkenden Rastung (9, 82) ist, wobei die Verkantungsöffnung oder die Rastung (9, 82) mit dem Hebel (2) verbunden ist oder die Verkantungsöffnung oder die Rastung (9, 82) mit dem Stift (52) verbunden ist, insbesondere die Verkantungsöffnung oder die Rastung mit dem Stift (52) über einen Hebel (2) verbunden ist.

10. Vorrichtung (1) nach einem der vorangehenden Ansprüche, **dadurch gekennzeichnet, dass**
in der Verbindung (14) zwischen dem Mischraum (12, 72) und den Kartuschen (4, 92) oder den Anschlüssen für die Kartuschen (4, 92) zumindest ein manuell zu öffnendes Ventilelement (18) angeordnet ist oder manuell zu öffnende Verschlüsse angeordnet sind.

11. Vorrichtung (1) nach einem der vorangehenden Ansprüche, **dadurch gekennzeichnet, dass**
die Vorrichtung (1) einen Griff (3) zum Halten der Vorrichtung (1) mit einer Hand aufweist, wobei der Hebel (2) oder der Stift (52) oder ein Bedienungselement (58) zur manuellen Bedienung des Hebels (2) oder des Stifts (52) derart im Bereich des Griffs (3) angeordnet ist, dass der Hebel (2) oder der Stift (52) oder das Bedienungselement (58) mit der gleichen Hand bedienbar ist, mit der die Vorrichtung (1) am Griff (3) gehalten wird.

12. Vorrichtung (1) nach einem der vorangehenden Ansprüche, **dadurch gekennzeichnet, dass**
der Mischraum (12, 72) auf der der Verbindung (14) zu den Kartuschen (4, 92) gegenüberliegenden Seite eine Austragsöffnung zum Applizieren des Mischguts aufweist, wobei bevorzugt an der Austragsöffnung ein Applikationsrohr (16) angeschlossen oder anschließbar ist.

13. Vorrichtung (1) nach einem der vorangehenden Ansprüche, **dadurch gekennzeichnet, dass**
in dem Mischraum (12, 72) statische Scherelemente (49, 76) zum Durchmischen des Mischguts angeordnet sind, wobei bevorzugt die Scherelemente (49, 76) den Fluss des Mischguts durch den Mischraum (12, 72) zerschneiden und/oder die Scherelemente (49, 76) in axialer Richtung, bezogen auf die Achse, um die sich die Mischflügel (46, 74) drehen, zwischen den Mischflügeln (46, 74) angeordnet sind.

14. Vorrichtung (1) nach einem der vorangehenden Ansprüche, **dadurch gekennzeichnet, dass**
an dem Hebel (2) ein Kreisbogen oder ein Kreisbogenabschnitt (10) angeordnet ist, auf dessen Stirnseite oder auf dessen Seitenfläche eine Antriebszahnreihe oder eine Reibfläche angeordnet ist, mit der ein Zahnrad (34) oder Reibrad des Getriebes antreibbar ist oder an dem Stift (52) eine Zahnung (60) oder eine Reibfläche angeordnet ist, mit der ein Zahnrad (62) oder Reibrad des Getriebes antreibbar ist.

15. Vorrichtung (1) nach einem der vorangehenden Ansprüche, **dadurch gekennzeichnet, dass**
das Getriebe eine derartige Übersetzung aufweist, dass bei einer vollständigen Bewegung des Hebels (2) oder des Stifts (52) die Mischflügel (46, 74) wenigstens 2 Umdrehungen im Mischraum (12, 72) vollführen, bevorzugt zwischen 2 und 10 Umdrehungen im Mischraum (12, 72) vollführen, besonders bevorzugt zwischen 2,5 und 5 Umdrehungen im Mischraum (12, 72) vollführen.

16. Vorrichtung (1) nach einem der vorangehenden Ansprüche, **dadurch gekennzeichnet, dass**
die Einrichtung zum Vortreiben der Förderkolben (32, 90) in den Kartuschen (4, 92) derart mit dem Hebel (2) oder dem Stift (52) verbunden ist, dass bei einer vollständigen Bewegung des Hebels (2) oder des Stifts (52) die Förderkolben (32, 90) um wenigstens 1 mm in den Kartuschen (4, 92) vorgetrieben werden, bevorzugt zwischen 2 mm und 12 mm in den Kartuschen (4, 92) vorgetrieben werden, besonders bevorzugt zwischen 5 mm und 7 mm in den Kartuschen (4, 92) vorgetrieben werden.

17. Vorrichtung (1) nach einem der vorangehenden Ansprüche, **dadurch gekennzeichnet, dass**
die Einrichtung zum Vortreiben der Förderkolben (32, 90) in den Kartuschen (4, 92) derart mit dem Hebel (2) oder dem Stift (52) verbunden ist, dass bei 5 bis 25 vollständigen Bewegungen des Hebels (2) oder des Stifts (52) die Förderkolben (32, 90) die Ausgangskomponenten zu wenigstens 90% aus den Kartuschen (4, 92) austreiben, bevorzugt dass bei 10 bis 20 vollständigen Bewegungen des Hebels (2) oder des Stifts (52) die Förderkolben (32, 90) die Ausgangskomponenten zu wenigstens 90% aus den Kartuschen (4, 92) austreiben.

18. Verfahren zum Mischen von fluiden Ausgangskomponenten, insbesondere zum Mischen eines medizinischen Zements, vorzugsweise mit einer Vorrichtung (1) nach einem der vorangehenden Ansprüche, wobei ein Hebel (2) gekippt wird oder ein Stift (52) in Längsrichtung bewegt wird, durch die Kraft der Bewegung des Hebels (2) oder des Stifts (52) Förderkolben (32, 90) in wenigstens zwei Kartuschen (4, 92) vorangetrieben werden und die in den Kartuschen (4, 92) enthaltenen Ausgangskomponenten mit Hilfe der Förderkolben (32, 90) in einen Mischraum (12, 72) gepresst werden, und
durch die Kraft der Bewegung des Hebels (2) oder des Stifts (52) Mischflügel (46, 74) in dem Mischraum (12, 72) gedreht werden, wobei die Übertragung der Kraft des Hebels (2) oder des Stifts (52) zur Drehung der Mischflügel (46, 74) über ein Getriebe erfolgt, wobei
nach der Bewegung des Hebels (2) oder des Stifts (52) dieser durch ein Rückstellelement (54) wieder in die Ausgangsposition überführt wird, wobei bei dieser Überführung in die Ausgangsposition die Förderkolben (32, 90) nicht bewegt werden,
**dadurch gekennzeichnet, dass**
bei dieser Überführung in die Ausgangsposition die Mischflügel (46, 74) nicht gedreht werden.

19. Verfahren nach Anspruch 18, **dadurch gekennzeichnet, dass**
durch die Drehung der Mischflügel (46, 74) in dem Mischraum (12, 72) die Ausgangskomponenten in dem Mischraum (12, 72) durchmischt und in Richtung einer Austragsöffnung gefördert werden.

20. Verfahren nach einem der Ansprüche 18 oder 19, **dadurch gekennzeichnet, dass**
das Getriebe über einen Zahnkranz (10) oder Reibkranz am Hebel (2) oder über eine Zahnreihe (60) oder eine Reibfläche am Stift (52) angetrieben wird.

21. Verfahren nach einem der Ansprüche 18 bis 20, **dadurch gekennzeichnet, dass**
die Bewegung des Stifts (52) oder des Hebels (2) mehrmals wiederholt wird, bevorzugt zwischen 5 und 25 mal wiederholt wird, um die Kartuschen (4, 92) zu wenigstens 90% zu entleeren.

22. Verfahren nach einem der Ansprüche 18 bis 21, **dadurch gekennzeichnet, dass**
das Getriebe einen vollständigen Hub des Hebels (2) oder des Stifts (52) in wenigstens 2 Umdrehungen der Mischflügel (46, 74) in dem Mischraum (12, 72) übersetzt, bevorzugt in 2,5 bis 5 Umdrehungen der Mischflügel (46, 74) in dem Mischraum (12, 72) übersetzt.

## Claims

1. Manually drivable device (1) for the mixing of a pasty mixing ware from at least two fluid starting components, in particular for the mixing of a medical cement, comprising
at least two cartridges (4, 92) containing the fluid starting components or at least two connectors for at least two cartridges (4, 92) containing the fluid starting components, whereby feed plungers (32, 90) are provided in the cartridges (4, 92) for expelling the starting components from the cartridges (4, 92);
a mixing space (12, 72) for the mixing of the starting components that is connected to the cartridges (4, 92) or connectors for the cartridges (4, 92) such that the starting components are guided into the mixing space (12, 72) when the starting components are expelled from the cartridges (4, 92) by the feed plungers (32, 90), whereby mixing vanes (46, 74) are provided in the mixing space (12, 72) and are mounted in the mixing space (12, 72) such that they can rotate;
a lever (2) that can be rotated about a pivot point and can be operated manually for operation of the device (1) or a pin (52) that can be shifted in longitudinal direction and can be operated manually in order to operate the device (1);
a gear connected to the lever (2) or pin (52), whereby the gear is appropriately connected to the rotatable mixing vanes (46, 74) in the mixing space (12, 72) such that the mixing vanes (46, 74) can be rotated in the mixing space (12, 72) upon a motion of the lever (2) or pin (52) mediated by the gear; and
a facility for propelling the feed plungers (32, 90) in the cartridges (4, 92), appropriately connected to the lever (2) or pin (52) such that the feed plungers (32, 90) can be propelled in the cartridges (4, 92) upon a motion of the lever (2) or pin (52), such that the motion of the lever (2) or pin (52) drives the rotation of the mixing vanes (46, 74) in the mixing space (12, 72) and the motion of the feed plungers (32, 90) into the cartridges (4, 92)
**characterised in that** a clutch (42, 44, 64) is provided in the connection from the gear to the mixing vanes (46, 74) or in the gear, and enables a rotation of the mixing vanes (46, 74) in a direction of rotation and prevents or reduces said rotation in the opposite direction of rotation.

2. Device (1) according to claim 1, **characterised in that**
the mixing vanes (46, 74) are inclined consistently with respect to the axis about which the mixing vanes (46, 74) rotate in the mixing space (12, 72) such that the rotation of the mixing vanes (46, 74) in the mixing space (12, 72) causes the mixing ware to be propelled in the mixing space (12, 72), whereby the mixing vanes (46, 74) are preferred to be inclined with respect to the axis by the same angle.

3. Device (1) according to claim 1 or 2, **characterised in that**
the gear is force-locking and/or form-fitting.

4. Device (1) according to any one of the preceding claims, **characterised in that** the gear comprises at least one tooth lock washer, at least one friction disk, at least one cogwheel (10, 34, 36, 38, 39, 62, 65, 66, 67), at least one worm wheel and/or at least one friction wheel by means of which the force and/or torque is transmitted, whereby the gear preferably comprises at least one conical tooth lock washer, at least one conical friction disk, at least one conical cogwheel (39, 66, 67) and/or at least one friction wheel for changing the direction of rotation.

5. Device (1) according to any one of the preceding claims, **characterised in that** the gear is a cogwheel gear, a friction wheel gear, a conical wheel gear, a contrate gear, a worm gear or a combination of said gears, preferably additionally comprising a spur gear.

6. Device (1) according to any one of the preceding claims, **characterised in that** the mixing vanes (46, 74) are arranged on a rotary shaft (33, 68, 70) that is mounted such as to be rotatable, whereby the rotary shaft (33, 68, 70) extends out of the mixing space (12, 72), preferably extends out of the mixing space (12, 72) via a seal, and the rotary shaft (33, 68, 70) is connected to the gear such that the rotary shaft (33, 68, 70) with the mixing vanes (46, 74) can be rotated in the mixing space (12, 72) by means of a motion of the lever (2) or pin (52).

7. Device (1) according to any one of the preceding claims, **characterised in that** a clutch (42, 44, 64) is provided in the rotary shaft (33, 68, 70) mounted such as to be rotatable.

8. Device (1) according to any one of the preceding claims, **characterised in that** a free-wheel clutch (64) is provided on or in the gear or a sprung rotating clutch (42, 44) is provided in the connection from the gear to the mixing vanes (46, 74), in particular in the rotary shaft (33, 68, 70) mounted such as to be rotatable.

9. Device (1) according to any one of the preceding claims, **characterised in that** the facility for propelling the feed plungers (32, 90) in the cartridges (4, 92) is a clamp rod (28) having a tiltable jamming opening or a gear rack (8, 86) having a unidirectionally-acting snap-in locking means (9, 82), whereby the jamming opening or the snap-in locking means (9, 82) is connected to the lever (2) or the jamming opening or the snap-in locking means (9, 82) is connected to the pin (52), in particular the jamming opening or the snap-in locking means (9, 82) is connected to the pin (52) via a lever (2).

10. Device (1) according to any one of the preceding claims, **characterised in that** at least one manually-opening valve element (18) or manually-opening closures is/are arranged in the connection (14) between the mixing space (12, 72) and the cartridges (4, 92) or in the connectors for the cartridges (4, 92).

11. Device (1) according to any one of the preceding claims, **characterised in that** the device (1) comprises a handle (3) for holding the device (1) by one hand, whereby the lever (2) or the pin (52) or an operating element (58) for manual operation of the lever (2) or pin (52) is arranged appropriately in the region of the handle (3) such that the lever (2) or pin (52) or operating element (58) can be operated by the same hand that holds the device (1) by the handle (3).

12. Device (1) according to any one of the preceding claims, **characterised in that** the mixing space (12, 72) comprises a dispensing opening for application of the mixing ware on the side opposite from the connection (14) to the cartridges (4, 92), whereby it is preferred to have an application tube (16) connected or be connectable to the dispensing opening.

13. Device (1) according to any one of the preceding claims, **characterised in that** static shearing elements (49, 76) for mixing of the mixing ware are arranged in the mixing space (12, 72), whereby it is preferred that said shearing elements (49, 76) cut the flow of the mixing ware through the mixing space (12, 72) and/or that the shearing elements (49, 76) are arranged in axial direction relative to the axis about which the mixing vanes (46, 74) rotate, between the mixing vanes (46, 74).

14. Device (1) according to any one of the preceding claims, **characterised in that** a circular arc or circular arc section (10) is arranged on the lever (2) and has, arranged on its front face or its side surface, a driving row of cogs or a friction surface by means of which a cogwheel (34) or friction wheel of the gear can be driven or **in that** a serration (60) or a friction surface is arranged on the pin (52) by means of which a cogwheel (62) or friction wheel of the gear can be driven.

15. Device (1) according to any one of the preceding claims, **characterised in that** the gear comprises an appropriate transmission ratio such that, upon a full motion of the lever (2) or pin (52), the mixing vanes (46, 74) perform at least 2 turns in the mixing space (12, 72), preferably perform between 2 and 10 turns in the mixing space (12, 72), particularly preferably between 2.5 and 5 turns in the mixing space (12, 72).

16. Device (1) according to any one of the preceding claims, **characterised in that** the facility for propelling the feed plungers (32, 90) in the cartridges (4, 92) is appropriately connected to the lever (2) or pin (52) such that, upon a full motion of the lever (2) or pin (52), the feed plungers (32, 90) are propelled in the cartridges (4, 92) by at least 1 mm, preferably are propelled in the cartridges (4, 92) by between 2 mm and 12 mm, particularly preferably are propelled in the cartridges (4, 92) by between 5 mm and 7 mm.

17. Device (1) according to any one of the preceding claims, **characterised in that** the facility for propelling the feed plungers (32, 90) in the cartridges (4, 92) is appropriately connected to the lever (2) or pin (52) such that the feed plungers (32, 90) expel at least 90% of the starting components from the cartridges (4, 92) upon 5 to 25 full motions of the lever (2) or pin (52), preferably such that the feed plungers (32, 90) expel at least 90% of the starting components from the cartridges (4, 92) upon 10 to 20 full motions of the lever (2) or pin (52).

18. Method for the mixing of fluid starting components, in particular for the mixing of a medical cement, preferably by means of a device (1) according to any one of the preceding claims,
whereby a lever (2) is being tilted or a pin (52) is being moved in longitudinal direction;
the force of the motion of the lever (2) or pin (52) propels feed plungers (32, 90) in at least two cartridges (4, 92) and presses the starting components contained in the cartridges (4, 92) into a mixing space (12, 72) by means of the feed plungers (32, 90); and
the force [of the motion] of the lever (2) or pin (52) rotates mixing vanes (46, 74) in the mixing space (12, 72), whereby the transmission of the force of the lever (2) or pin (52) for rotation of the mixing vanes (46, 74) is effected by means of a gear, whereby
the lever (2) or pin (52), after being moved, is restored to the starting position by means of a restoring element (54), whereby the feed plungers (32, 90) are not moved during said restoring to the starting position,
**characterised in that**
the mixing vanes (46, 74) are not being rotated during said restoring to the starting position.

19. Method according to claim 18, **characterised in that**
the rotation of the mixing vanes (46, 74) in the mixing space (12, 72) mixes the starting components in the mixing space (12, 72) and conveys them in the direction of a dispensing opening.

20. Method according to any one of the claims 18 or 19, **characterised in that** the gear is driven by means of a sprocket wheel (10) or friction sprocket wheel on the lever (2) or a row of cogs (60) or a friction surface on the pin (52).

21. Method according to any one of the claims 18 to 20, **characterised in that** the motion of the pin (52) or lever (2) is repeated, preferably between 5 and 25 times, in order to empty the cartridges (4, 92) by at least 90%.

22. Method according to any one of the claims 18 to 21, **characterised in that** the gear transmits a full stroke of the lever (2) or pin (52) into at least 2 turns of the mixing vanes (46, 74) in the mixing space (12, 72), preferably into 2.5 to 5 turns of the mixing vanes (46, 74) in the mixing space (12, 72).

## Revendications

1. Dispositif (1) actionnable manuellement pour mélanger un produit à mélanger pâteux à base d'au moins deux composants de départ fluides, notamment pour mélanger un ciment à usage médical, présentant
au moins deux cartouches (4, 92) contenant les composants de départ fluides ou au moins deux raccordements d'au moins deux cartouches (4, 92) contenant les composants de départ fluides, sachant que des pistons de refoulement (32, 90) sont prévus dans lesdites cartouches (4, 92) pour expulser les composants de départ hors des cartouches (4, 92) ;
une chambre de mélange (12, 72) pour mélanger les composants de départ, qui est reliée avec les cartouches (4, 92) ou avec les raccordements des cartouches (4, 92), de sorte que les composants de départ sont acheminés dans ladite chambre de mélange (12, 72) lors de l'expulsion des composants de départ hors des cartouches (4, 92) à l'aide des pistons de refoulement (32, 90), sachant que des pales mélangeuses (46, 74) sont prévues dans ladite chambre de mélange (12, 72) et qu'elles sont montées dans ladite chambre de mélange (12, 72) de façon à pouvoir pivoter ;
un levier (2) actionnable manuellement et pouvant pivoter autour d'un point de rotation ou une tige (52) actionnable manuellement et déplaçable dans le sens longitudinal pour manoeuvrer le dispositif (1) ;
un mécanisme de transmission, qui est relié avec le levier (2) ou avec la tige (52), sachant que ledit mécanisme de transmission est raccordé aux pales mélangeuses rotatives (46, 74) dans la chambre de mélange (12, 72), de sorte que les pales mélangeuses (46, 74) peuvent pivoter dans la chambre de mélange (12, 72) moyennant le mouvement du levier (2) ou de la tige (52) relayé par ledit mécanisme de transmission et un équipement pour faire avancer les pistons de refoulement (32, 90) dans les cartouches, qui est relié avec le levier (2) ou avec la tige (52), de sorte que les pistons de refoulement (32, 90) doivent avancer dans les cartouches (4, 92) lors du mouvement du levier (2) ou de la tige (52), de sorte que le mouvement du levier (2) ou de la tige (52) entraîne la rotation des pales mélangeuses (46, 74) dans la chambre de mélange (12, 72) et le déplacement des pistons de refoulement (32, 90) dans les cartouches (4, 92),
**caractérisé en ce**
**qu'**un accouplement (42, 44, 64) est prévu dans la liaison du mécanisme de transmission avec les pales mélangeuses (46, 74) ou dans le mécanisme de transmission permettant le pivotement des pales mélangeuses (46, 74) dans un sens de rotation et l'empêchant ou le réduisant dans le sens de rotation opposé.

2. Dispositif (1) selon la revendication 1 **caractérisé en ce que**
les pales mélangeuses (46, 74) sont inclinées dans le même sens vers l'axe, autour duquel lesdites pales mélangeuses (46, 74) pivotent dans la chambre de mélange (12, 72), de sorte que l'avancement du produit à mélanger est assuré dans la chambre de mélange (12, 72) par la rotation desdites pales mélangeuses (46, 74), sachant que lesdites pales mélangeuses (46, 74) sont inclinées de préférence du même angle par rapport à l'axe.

3. Dispositif (1) selon les revendications 1 ou 2 **caractérisé en ce que**
le mécanisme de transmission agit par complémentarité de forme et/ou de force.

4. Dispositif (1) selon l'une des revendications précédentes, **caractérisé en ce que**
le mécanisme de transmission présente au moins une rondelle éventail, au moins un disque de friction, au moins un pignon (10, 34, 36, 38, 39, 62, 65, 66, 67), au moins une roue à denture hélicoïdale et/ou au moins une roue de friction, par lequel ou par laquelle la force ou bien le couple de rotation sont transmis, sachant que ledit mécanisme de transmission présente de préférence au moins une rondelle éventail conique, au moins un disque de friction conique, au moins un pignon conique (39, 66, 67) et/ou au moins une roue de friction pour modifier le sens de rotation.

5. Dispositif (1) selon l'une des revendications précédentes, **caractérisé en ce que**
le mécanisme de transmission est un engrenage, un engrenage à friction, un engrenage à roues coniques, un engrenage à couronne dentée, un engrenage à vis sans fin ou une combinaison de ces engrenages, présentant en plus de préférence un engrenage droit.

6. Dispositif (1) selon l'une des revendications précédentes, **caractérisé en ce que**
les pales mélangeuses (46, 74) sont disposées sur un axe de rotation (33, 68, 70) monté de façon à pouvoir pivoter, sachant que l'axe de rotation (33, 68, 70) s'étend au-delà de la chambre de mélange (12, 72), qu'il s'étend au-delà de la chambre de mélange (12, 72) de préférence par le biais d'un joint d'étanchéité et que l'axe de rotation (33, 68, 70) est relié avec le mécanisme de transmission, de sorte que l'axe de rotation (33, 68, 70) peut pivoter avec les pales mélangeuses (46, 74) dans la chambre de mélange (12, 72) moyennant un mouvement du levier (2) ou de la tige (52).

7. Dispositif (1) selon l'une des revendications précédentes, **caractérisé en ce que**
l'accouplement (42, 44, 64) est prévu dans l'axe de rotation (33, 68, 70) monté de façon à pouvoir pivoter.

8. Dispositif (1) selon l'une des revendications précédentes, **caractérisé en ce qu'**un
accouplement à roue libre (64) est prévu sur le mécanisme de transmission ou dans le mécanisme de transmission ou qu'un accouplement rotatif à ressort (42, 44) est prévu dans la liaison du mécanisme de transmission avec les pales mélangeuses (46, 74), notamment dans l'axe de rotation (33, 68, 70) monté de façon à pouvoir pivoter.

9. Dispositif (1) selon l'une des revendications précédentes, **caractérisé en ce que**
l'équipement servant à faire avancer les pistons de refoulement (32, 90) dans les cartouches (4, 92) est une tige de serrage (28) à ouverture de calage basculante ou une crémaillère (8, 86) à encliquetage unidirectionnel (9, 82), sachant que l'ouverture de calage ou l'encliquetage (9, 82) sont reliés avec le levier (2) ou que l'ouverture de calage ou l'encliquetage (9, 82) sont reliés avec la tige (52), notamment que l'ouverture de calage ou l'encliquetage (9, 82) sont reliés avec la tige (52) par l'intermédiaire du levier (2).

10. Dispositif (1) selon l'une des revendications précédentes, **caractérisé en ce qu'**au
moins un élément de valve (18) à ouvrir manuellement est disposé ou que des obturateurs à ouvrir manuellement sont disposés dans la liaison (14) entre la chambre de mélange (12, 72) et les cartouches (4, 92) ou entre les raccordements des cartouches (4, 92).

11. Dispositif (1) selon l'une des revendications précédentes, **caractérisé en ce que**
le dispositif (1) présente une poignée (3) pour tenir le dispositif (1) d'une main, sachant que le levier (2) ou la tige (52) ou un élément de commande (58) pour manoeuvrer manuellement le levier (2) ou la tige (52) sont disposés au niveau de la poignée (3), de telle manière que le levier (2) ou la tige (52) ou l'élément de commande (58) sont actionnables avec la même main que celle servant à tenir le dispositif (1) par la poignée (3).

12. Dispositif (1) selon l'une des revendications précédentes, **caractérisé en ce que**
la chambre de mélange (12, 72) présente un orifice de distribution pour appliquer le produit à mélanger du côté opposé à la liaison (14) avec les cartouches (4, 92), sachant qu'un tube d'application (18) est raccordé ou raccordable de préférence à l'orifice de distribution.

13. Dispositif (1) selon l'une des revendications précédentes, **caractérisé en ce que**
des éléments de cisaillement statiques (49, 76) pour brasser le produit à mélanger sont disposés dans la chambre de mélange (12, 72), sachant que les éléments de cisaillement (49, 76) coupent de préférence le flux du produit à mélanger à travers la chambre de mélange (12, 72) et/ou que les éléments de cisaillement (49, 76) sont disposés entre les pales mélangeuses (46, 74) dans le sens axial par rapport à l'axe, autour duquel les pales mélangeuses (46, 74) pivotent.

14. Dispositif (1) selon l'une des revendications précédentes, **caractérisé en ce qu'**un
arc de cercle ou qu'une section d'arc de cercle (10) sont disposés près du levier (2), sur la face frontale ou sur la face latérale duquel une série de pignons d'entraînement ou une surface de frottement sont disposés, avec laquelle un pignon (34) ou une roue de friction du mécanisme de transmission sont actionnables ou une denture (60) ou une surface de frottement sont disposées sur la tige (52), avec laquelle un pignon (34) ou une roue de friction du mécanisme de transmission sont actionnables.

15. Dispositif (1) selon l'une des revendications précédentes, **caractérisé en ce que**
le mécanisme de transmission présente un tel rapport de transmission que les pales mélangeuses (46, 74) exécutent au moins 2 tours dans la chambre de mélange (12, 72), de préférence entre 2 et 10 tours dans la chambre de mélange (12, 72), mieux encore entre 2,5 et 5 tours dans la chambre de mélange (12, 72) lors du déplacement complet du levier (2) ou de la tige (52).

16. Dispositif (1) selon l'une des revendications précédentes, **caractérisé en ce que**
l'équipement servant à faire avancer les pistons de refoulement (32, 90) dans les cartouches (4, 92) est relié avec le levier (2) ou la tige (52), de manière à ce que les pistons de refoulement (32, 90) sont avancés d'au moins 1 mm dans les cartouches (4, 92), de préférence entre 2 mm et 12 mm dans les cartouches (4, 92), mieux encore entre 5 mm et 7 mm dans les cartouches (4, 92) lors du déplacement complet du levier (2) ou de la tige (52).

17. Dispositif (1) selon l'une des revendications précédentes, **caractérisé en ce que**
l'équipement servant à faire avancer les pistons de refoulement (32, 90) dans les cartouches (4, 92) est relié avec le levier (2) ou la tige (52), de manière à ce que les pistons de refoulement (32, 90) expulsent les composants de départ à raison d'au moins 90% hors des cartouches (4, 92) lors de 5 à 25 déplacements complets du levier (2) ou de la tige (52), de préférence que les pistons de refoulement (32, 90) expulsent les composants de départ à raison d'au moins 90% hors des cartouches (4, 92) lors de 10 à 20 déplacements complets du levier (2) ou de la tige (52).

18. Procédé pour mélanger des composants de départ fluides, notamment pour mélanger un ciment à usage médical, de préférence à l'aide d'un dispositif (1) selon l'une des revendications précédentes, sachant qu'un levier (2) est incliné ou qu'une tige (52) est déplacée dans le sens longitudinal, que des pistons de refoulement (32, 90) sont avancés dans au moins deux cartouches (4, 92) par la force de déplacement du levier (2) ou de la tige (52) et que les composants de départ contenus dans les cartouches (4, 92) sont comprimés dans une chambre de mélange (12, 72) à l'aide des pistons de refoulement (32, 90) et que
des pales mélangeuses (46, 74) sont mises en rotation dans la chambre de mélange (12, 72)) par la force de déplacement du levier (2) ou de la tige (52), sachant que le transfert de la force du levier (2) ou de la tige (52) pour faire pivoter les pales mélangeuses (46, 74) est assuré par un mécanisme de transmission, sachant
qu'après le déplacement du levier (2) ou de la tige (52), ces derniers sont ramenés à la position initiale par un élément de rappel (54), sachant que les pistons de refoulement (32, 90) ne sont pas déplacés lors de ce retour à la position initiale,
**caractérisé en ce que**
les pales mélangeuses (46, 74) ne sont pas mises en rotation lors de ce retour à la position initiale.

19. Procédé selon la revendication 18, **caractérisé en ce que**
les composants de départ sont brassés dans la chambre de mélange (12, 72) et acheminés vers un orifice de distribution par la rotation des pales mélangeuses (46, 74) dans la chambre de mélange (12, 72).

20. Procédé selon l'une des revendications 18 ou 19, **caractérisé en ce que**
le mécanisme de transmission est actionné au moyen d'une couronne dentée (10) ou d'une couronne à entraînement par friction sur le levier (2) ou par le biais d'une série de dents (60) ou d'une surface de frottement sur la tige (52).

21. Procédé selon l'une des revendications de 18 à 20, **caractérisé en ce que**
le déplacement de la tige (52) ou du levier (2) est répété plusieurs fois, de préférence de 5 à 25 fois pour vider les cartouches (4, 92) d'au moins 90%.

22. Procédé selon l'une des revendications de 18 à 21, **caractérisé en ce que**
le mécanisme de transmission convertit une course complète du levier (2) ou de la tige (52) en au moins 2 tours des pales mélangeuses (46, 74) dans la chambre de mélange (12, 72), de préférence selon un rapport de 2,5 à 5 tours des pales mélangeuses (46, 74) dans la chambre de mélange (12, 72).
